# EUROPEAN PATENT APPLICATION

(11) **EP 4 596 709 A1**
(43) Date of publication of application: **06.08.2025**
(21) Application number: 23870901.8
(22) Date of filing: 27.09.2023
(51) Int. Cl.: C12Q 1/68

(54) **SEQUENCING METHOD**

(30) Priority: 28.09.2022 WO PCT/CN2022/122149
(71) Applicant: MGI Tech Co., Ltd., Shenzhen, Guangdong 518083 (CN)
(72) Inventor: YANG, Jin, Shenzhen, Guangdong 518083 (CN); SONG, Chongyang, Shenzhen, Guangdong 518083 (CN); XING, Chengmei, Shenzhen, Guangdong 518083 (CN); XU, Chongjun, Shenzhen, Guangdong 518083 (CN)
(74) Representative: Hoffmann Eitle
(86) International application number: PCT/CN2023/121926
(87) International publication number: WO 2024/067674

(57) **Abstract**

Provided is a hydrogel-based sequencing method suitable for a flowcell sequencing system and also supporting open-source sequencing system, and which can effectively reduce costs while ensuring sequencing efficiency and accuracy.

## Description

### Technical Field

The present invention relates to the field of sequencing. Specifically, the present invention provides a hydrogel-based sequencing method, which is applicable to a Flowcell sequencing system and also supports an open sequencing system, and can effectively reduce costs while ensuring sequencing efficiency and accuracy.

### Background Art

With the development of sequencing technology and the improvement of social awareness, high-throughput gene sequencing has been widely used in various fields such as scientific research, precision medicine, social health and public safety. In recent years, although the cost of sequencing has continued to decline, the price of each WGS (whole genome sequencing, 100Gb data) of more than 1,000 RMB still hinders the wider application of gene sequencing. In the existing high-throughput sequencing (here mainly refers to massively parallel sequencing, MPS) technical solutions, two technical solutions are mainly used to achieve liquid exchange and reaction. The first is the Flowcell solution, which uses syringe pumps, rotary valves, etc. to extract reagents from different positions, inject them into the flow channel of chip, push away the old reagents, replace them with new reagents, thereby completing the liquid exchange, and allowing the replaced active ingredients to undergo biochemical reactions; the second is the Dip sequencing (DIP) solution, which pumps reagents into reagent tanks, and transfers the sequencing chip between different reagent tanks, whenever the chip is dipped in a new reagent, the liquid and active ingredients are exchanged through diffusion. In the Flowcell solution, in order to achieve effective replacement of reagents, the replacement ratio (that is, the ratio of the volume of the new reagent pumped in to completely replace the old reagent in the flow channel to the flow channel volume) is relatively high, and generally reached 2~3 or more, and the reagents are disposable, so although the reaction efficiency is high, the cost is still relatively high. In the Dip sequencing solution, the sequencing reagents can be reused, so the cost of reagents can be effectively reduced; however, the Dip sequencing solution has the problem that the chip is easy to dry during the sequencing process, the use of the Dip sequencing solution in some steps will increase the cost or affect the quality, and the reuse of reagents has the risk of affecting the quality.

Therefore, ensuring sequencing efficiency and accuracy while effectively reducing costs is a great challenge for high-throughput sequencing and a problem that needs to be solved urgently.

### Contents of the Invention

The present application provides a hydrogel-based sequencing method, which not only supports traditional sequencing systems based on Flowcells, but also supports open sequencing systems and large-size sequencing chips. The method can reduce reagent loss and avoid drying and crystallization damage to target nucleic acid molecules on the surface of slide; and does not require complex fluids and temperature control; and can also effectively reduce the amount of reagents used, thereby achieving single use and reducing costs.

In one aspect, the present invention provides a method for sequencing a target nucleic acid molecule, which comprises the following steps: (1) contacting nucleotides having a label with the target nucleic acid molecule to allow the incorporation of a nucleotide; (2) detecting the label of the nucleotide as incorporated; (3) removing the label; wherein at least one of steps (1) and (3) is performed in gel state; optionally, the method comprises repeating the above steps in sequence to determine the sequence of the target nucleic acid molecule.

In certain embodiments, the incorporation of the nucleotide described in step (1) results in formation of a phosphodiester bond. In some embodiments, step (3) allows a complementary strand of the target nucleic acid molecule to undergo an extension reaction.

In some embodiments, the label is a fluorescent label.

In some embodiments, the label is an affinity label, and step (2) comprises step (2a), adding a luciferase carrying an affinity label to perform an affinity reaction with the product of step (1); step (2b), adding a specific substrate to perform a catalytic reaction with the product of step (2a); and step (2c), detecting the product of step (2b). In some embodiments, steps (2a) and (2b) are performed in gel state.

In some embodiments, step (3) is performed in gel state, it comprises the following steps: (3a) contacting a reaction composition comprising a removal reagent with the product of the previous step, in which the reaction composition further comprises a polymer having reversible thermogel property, and the reaction composition is in liquid state; (3b) adjusting the reaction composition to a preset temperature to convert the reaction composition into gel state, and performing an excision reaction to allow the removal of the label on the nucleotide; (3c) adjusting the temperature of the reaction composition to convert the reaction composition into liquid state, and removing the reaction composition.

On the other hand, the present invention provides a method for sequencing a target nucleic acid molecule, comprising the following steps: (1) contacting nucleotides with the target nucleic acid molecule to allow the incorporation of a nucleotide, wherein the nucleotides carry a blocking group; (2) detecting the nucleotide as incorporated; (3) removing the blocking group; wherein at least one of steps (1) and (2) is performed in gel state; optionally, the method comprises repeating the above steps in sequence to determine the sequence of the target nucleic acid molecule. Preferably, the incorporation of the nucleotide in step (1) results in formation of a phosphodiester bond. Preferably, step (3) allows the complementary chain of the target nucleic acid molecule to undergo an extension reaction.

In certain embodiments, step (2) comprises step (2a), adding an affinity reagent carrying a fluorescent label to perform an affinity reaction with the product of step (1); and step (2b), detecting the product of step (2a). Preferably, step (2a) is performed in gel state. Preferably, the affinity reagent is an antibody against a nucleotide A\T\C\G\U carrying a blocking group, and the antibody can specifically recognize the nucleotide and undergo an affinity reaction therewith.

In some embodiments, step (3) is performed in gel state and comprises the following steps: (3a) contacting a reaction composition comprising a removal agent with the product of the previous step, wherein the reaction composition further comprises a polymer having reversible thermogel property, and the reaction composition is in liquid state; (3b) adjusting the reaction composition to a preset temperature to convert the reaction composition into gel state, and performing an excision reaction to allow the removal of the blocking group on the nucleotide; (3c) adjusting the temperature of the reaction composition to convert the reaction composition into liquid state, and removing the reaction composition.

On the other hand, the present invention provides a method for sequencing a target nucleic acid molecule, which comprises the following steps: (1) contacting nucleotides having a label with the target nucleic acid molecule to allow the incorporation of a nucleotide; (2) detecting the label of the nucleotide as incorporated; (3) removing the nucleotide as incorporated and the label it carried; (4) contacting unlabeled nucleotides with the target nucleic acid molecule to allow the incorporation of an unlabeled nucleotide; wherein at least one of steps (1), (3) and (4) is performed in gel state; optionally, the method comprises repeating the above steps in sequence to determine the sequence of the target nucleic acid molecule. In some embodiments, the label is a fluorescent label.

In some embodiments, in step (1), the nucleotide is incorporated in accordance with only the principle of base complementary pairing and binds to the 3' end of the complementary chain of the target nucleic acid without forming a phosphodiester bond. In some embodiments, in step (4), the nucleotide is incorporated by a polymerization reaction to form a phosphodiester bond. In some embodiments, in step (4), the nucleotide incorporated has a blocking group. In some embodiments, step (3) further comprises removing the blocking group left at the 3' end of the target nucleic acid complementary chain, which will allow the extension reaction of the target nucleic acid molecule complementary chain.

In some embodiments, step (3) is performed in solution state, it comprises: removing the incorporated labeled nucleotide under appropriate conditions using an elution reagent; preferably, it further comprises removing the remaining blocking group.

In certain embodiments, step (3) is performed in gel state, it comprises the following steps: (3a) contacting a reaction composition comprising an elution reagent with the product of the previous step, wherein the reaction composition further comprises a polymer having reversible thermogel property, and the reaction composition is in liquid state; (3b) adjusting the reaction composition to a preset temperature to convert the reaction composition into gel state, and performing an elution reaction to allow the removal of the nucleotide as incorporated and the removal of the carried label; (3c) adjusting the temperature of the reaction composition to convert the reaction composition into liquid state, and removing the reaction composition. In such embodiments, the method may further comprises, for example, removing the blocking group after the incorporation reaction of step (4), and then performing the nucleotide incorporation of step (1) in the next round of reaction.

In some embodiments, step (4) is performed in gel state, it comprises the following steps: (4a) contacting a reaction composition comprising unlabeled nucleotides with the target nucleic acid molecule, wherein the reaction composition comprises a polymer having reversible thermogel property, and the reaction composition is in liquid state; preferably, the unlabeled nucleotides have a blocking group; (4b) adjusting the reaction composition to a preset temperature to convert the reaction composition into gel state, and performing a polymerization reaction to allow the incorporation of nucleotide; (4c) adjusting the temperature of the reaction composition to convert the reaction composition into liquid state, and optionally removing the reaction composition.

In some embodiments, in any of the above aspects, step (1) is performed in gel state, it comprises the following steps: (1a) contacting a reaction composition comprising nucleotides with the target nucleic acid molecule, wherein the nucleotides are labeled with a label or carry a blocking group, the reaction composition comprises a polymer having reversible thermogel property, and the reaction composition is in liquid state; (1b) adjusting the reaction composition to a preset temperature to convert the reaction composition into gel state, and performing an incorporation reaction to allow the incorporation of a nucleotide; (1c) adjusting the temperature of the reaction composition to convert the reaction composition into liquid state. In some embodiments, after converting the reaction composition into liquid state in step (1c), it further comprises: removing the reaction composition and then perform the detection reaction of step (2) or directly performing the detection reaction of step (2).

In some embodiments, in any of the above aspects, the labeled nucleotide described in step (1) further comprises an independent blocking group; or, the label contained in the labeled nucleotides can be used as a blocking group.

In some embodiments, in any of the above aspects, before, during or after any step before step (3) (for example, after step (1) and before step (2)), the method further comprises: a step of contacting unlabeled nucleotides with the target nucleic acid molecule, wherein the unlabeled nucleotides carry a blocking group; and the step is optionally performed in gel state. In some embodiments, after step (1) and before step (2), the method comprises the following steps: (i) contacting a reaction composition comprising unlabeled nucleotides with the target nucleic acid molecule, wherein the unlabeled nucleotides carry a blocking group, the reaction composition comprises a polymer having reversible thermogel property, and the reaction composition is in liquid state; (ii) adjusting the reaction composition to a preset temperature to convert the reaction composition into gel state, and performing an incorporation reaction to allow the incorporation of nucleotide; (iii) adjusting the temperature of the reaction composition to convert the reaction composition into liquid state, and removing the reaction composition.

In some embodiments, in any of the above aspects, before step (1), the method further comprises: a step of contacting a sequencing primer with the target nucleic acid molecule to allow hybridization; the step is optionally performed in gel state. Preferably, before step (1), the method comprises the following steps: (i) contacting a reaction composition comprising a sequencing primer with the target nucleic acid molecule, wherein the reaction composition comprises a polymer having reversible thermogel property, and the reaction composition is in liquid state; (ii) adjusting the reaction composition toa preset temperature to convert the reaction composition into gel state so that the sequencing primer hybridizes to the target nucleic acid molecule; preferably, the preset temperature allows the primer hybridization to occur; (iii) adjusting the temperature of the reaction composition to convert the reaction composition into liquid state, and removing the reaction composition.

In certain embodiments, in any of the above aspects, the polymer having reversible thermogel property responds to a temperature change by changing from liquid state to gel state when the temperature rises. Preferably, the concentration of the polymer is about 0.5~30% (w/w), such as about 10~30% (w/w), about 15~25% (w/w), about 18~22% (w/w), such as about 20% (w/w). Preferably, the polymer has a gelling concentration of about 0.5% to 25% (w/w). Preferably, the polymer has a gelling temperature of about 10~65°C. Preferably, the polymer is a block copolymer, a graft copolymer or a homopolymer. Preferably, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxy polyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA).

In certain embodiments, in any of the above aspects, (a) the temperature at which the gel state is converted into the liquid state is about 0~30°C (e.g., about 0~10°C, such as about 2~8°C, such as about 4°C; such as about 20~30°C, such as about 20~25°C, such as about 20°C or about 25°C); (b) the preset temperature at which the reaction composition comprising nucleotides (e.g., labeled nucleotides, unlabeled nucleotides, or unlabeled nucleotides with a blocking group) is converted into gel state is about 50~60°C, such as about 55~60°C, such as about 55°C; (c) the preset temperature at which the reaction composition comprising a removal reagent or an elution reagent is converted into gel state is about 50~60°C, such as about 55~60°C, such as about 55°C; and/or, (d) the preset temperature at which the reaction composition comprising a sequencing primer is converted into gel state is about 35~45°C, such as about 37~45°C, about 37~42°C, such as about 37°C.

In some embodiments, in any of the above aspects, the target nucleic acid molecule is fixed to a solid phase support, such as a chip.

In some embodiments, in any of the above aspects, the method is an open sequencing method. Preferably, the target nucleic acid molecule is fixed to the surface of an open sequencing slide. Preferably, the method comprises allowing the open sequencing slide with the target nucleic acid molecule fixed thereon to contact with or dip into one or more sequencing reagents required for the sequencing reaction, respectively, and the one or more sequencing reagents are each placed in a separate reaction container.

In some embodiments, in any of the above aspects, the method is a Flowcell sequencing method. Preferably, the target nucleic acid molecule is fixed to the surface of a flow cell. Preferably, the method comprises introducing one or more sequencing reagents required for the sequencing reaction into a flow cell with the target nucleic acid molecule fixed thereon.

In another aspect, the present invention provides a kit, comprising: (a) a composition comprising labeled nucleotides and a polymer having reversible thermogel property; and/or, (b) a composition comprising a removal agent and a polymer having reversible thermogel property. In certain embodiments, the kit further comprises: (c) a composition comprising unlabeled nucleotides and a polymer having reversible thermogel property, wherein the unlabeled nucleotides carry a blocking group. In certain embodiments, the kit further comprises: (d) a composition comprising a sequencing primer and a polymer having reversible thermogel property. In certain embodiments, the polymer having reversible thermogel property described in any one of (a) to (d) may be the same or different. In certain embodiments, the kit further comprises an additional reagent required for sequencing, such as a washing solution.

On the other hand, the present invention provides a kit, comprising: (1) a composition comprising nucleotides and a polymer having reversible thermogel property, wherein the nucleotides carry a blocking group; and/or, (2) a composition comprising an affinity reagent carrying a fluorescent label and a polymer having reversible thermogel property. In certain embodiments, the kit further comprises: (3) a composition comprising a removal reagent and a polymer having reversible thermogel property. In certain embodiments, the kit further comprises: (4) a composition comprising a sequencing primer and a polymer having reversible thermogel property. In certain embodiments, the polymer having reversible thermogel property described in any one of (1) to (4) may be the same or different. In certain embodiments, the kit further comprises an additional reagent required for sequencing, such as a washing solution.

On the other hand, the present invention provides a kit, comprising: (i) a composition comprising labeled nucleotides and a polymer having reversible thermogel property; (ii) a composition comprising an elution reagent and a polymer having reversible thermogel property; and/or, (iii) a composition comprising unlabeled nucleotides and a polymer having reversible thermogel property; preferably, the unlabeled nucleotides carry a blocking group. In certain embodiments, the kit further comprises: (iv) a composition comprising unlabeled nucleotides and a polymer having reversible thermogel property, wherein the unlabeled nucleotides carry a blocking group. In certain embodiments, the kit further comprises: (v) a composition comprising a sequencing primer and a polymer having reversible thermogel property. In certain embodiments, the polymer having reversible thermogel property described in any one of (i) to (v) may be the same or different. In certain embodiments, the kit further comprises an additional reagent required for sequencing, such as a washing solution.

In certain embodiments, in any of the above aspects, the polymer having reversible thermogel property responds to a temperature change by changing from liquid to gel when the temperature rises. Preferably, the concentration of the polymer is about 0.5~30% (w/w), such as about 10~30% (w/w), about 15~25% (w/w), about 18~22% (w/w), such as about 20% (w/w). Preferably, the polymer has a gelling concentration of about 0.5% to 25%. Preferably, the polymer has a gelling temperature of about 10~65°C. Preferably, the polymer is a block copolymer, a graft copolymer or a homopolymer. Preferably, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxy polyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA).

In certain embodiments, in any of the above aspects, the temperature at which the composition described in any of (a) to (d), (1) to (4), (i) to (v) is converted from gel state to liquid state is about 0~30°C (e.g., about 0~10°C, such as about 2~8°C, such as about 4°C; such as about 20~30°C, such as about 20~25°C, such as about 20°C or about 25°C); the preset temperature at which the reaction composition described in (a), (1) or (i) is converted into gel state is about 50~60°C, such as about 55~60°C, such as about 55°C; the preset temperature at which the reaction composition described in (2) is converted into gel state is about 30~40°C, such as about 30~37°C, about 32~37°C, about 35~37°C, such as about 35°C; the preset temperature at which the reaction composition described in (b), (3) or (ii) is converted into gel state is about 50~60°C, such as about 55~60°C, such as about 55°C; the preset temperature at which the reaction composition described in (iii) is converted into gel state is about 50~60°C, such as about 55~60°C, such as about 55°C; the preset temperature at which the reaction composition described in (c) or (iv) is converted into gel state is about 50~60°C, such as about 55~60°C, such as about 55°C; and/or, the preset temperature at which the reaction composition described in (d), (4) or (v) is converted into gel state is about 35~45°C, such as about 37~45°C, about 37~42°C, such as about 37°C. The temperature ranges of the above embodiments do not constitute a specific limitation on the temperature setting of the present invention. The temperature setting is based on the actual temperature required by the reaction reagent. For example, if the suitable reaction temperature for the polymerase incorporated into the reaction is 37°C, the gel temperature of step (a) is set to 37°C; if the temperature of primer hybridization is 60°C, the temperature of the gel in the corresponding step is set to 60°C, etc.

The embodiments of the present invention will be described in detail below in conjunction with the accompanying drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows the states of reagents containing 20% PF127 or 1% Methocel A15C at different temperatures.
Fig. 2 shows the cutting effect of the RR-gel reagent in Example 1.
Fig. 3 shows the Barcode sequencing of the RR-gel reagent in Example 1.
Fig. 4 shows the Read1 sequencing of the Hot-gel reagent in Example 2.
Fig. 5 shows the Barcode sequencing of the Hot-gel, Cold-gel and RR-gel reagents in Example 3.
Fig. 6 shows the results of Read1 sequencing of Hot-gel, Cold-gel and RR-gel reagents in Example 4.
Fig. 7 shows the primer hybridization reaction of Primer-gel in Example 5.

### Detailed Description of the invention

The present invention provides a hydrogel-based sequencing method and a kit for the sequencing method.

Unless otherwise defined, all technical and scientific terms used herein have the same meaning as commonly understood by a person of ordinary skill in the art to which the present invention belongs. All publications mentioned herein are incorporated herein by reference.

Where a numerical range is provided, it should be understood that each intermediate value (e.g., any integer value) between the upper and lower limits of the range is included within the scope of the present invention. When the term "about" is used to describe a measurable value (e.g., concentration, temperature, etc.), it means a range of ±10%, ±5%, ±2% or ±1% of a given value.

### Sequencing method

In a first aspect, the present invention provides a method for sequencing a target nucleic acid molecule, comprising the following steps:
(1) contacting nucleotides having a label with the target nucleic acid molecule to allow the incorporation of a nucleotide;
(2) detecting the label of the nucleotide as incorporated;
(3) removing the label.

According to one embodiment of the present invention, step (1) is performed in gel state.

According to another embodiment of the present invention, steps (1) and (3) are performed in gel state.

As used herein, the expression "performing in gel state" means that the reaction mixture is in gel state. In certain embodiments, the method comprises repeating the above steps in sequence to determine the sequence of the target nucleic acid molecule. In certain embodiments, the nucleotide described in step (1) is incorporated to form a phosphodiester bond. It is understood by those skilled in the art that further extension of the complementary chain of the target nucleic acid molecule will be allowed after step (3).

In certain embodiments, the label is a fluorescent label.

In certain embodiments, the label is an affinity label, and step (2) comprises step (2a), adding a luciferase carrying an affinity label to perform an affinity reaction with the product of step (1); step (2b), adding a specific substrate to perform a catalytic reaction with the product of step (2a); and step (2c), detecting the product of step (2b).

In certain embodiments, steps (2a) and (2b) are performed in gel state.

In some embodiments, step (2a) is performed in gel state, it comprises the following steps: (i) contacting a reaction composition comprising a luciferase carrying an affinity label with the product of the previous step, wherein the reaction composition further comprises a polymer having reversible thermogel property, and the reaction composition is in liquid state; (ii) adjusting the reaction composition to a preset temperature to convert the reaction composition into gel state, and performing an affinity reaction; (iii) adjusting the temperature of the reaction composition to convert the reaction composition into liquid state, and removing the reaction composition.

In some embodiments, step (2b) is performed in gel state, it comprises the following steps: (i) contacting a reaction composition comprising a specific substrate with the product of the previous step, wherein the reaction composition further comprises a polymer having reversible thermogel property, and the reaction composition is in a liquid state; (ii) adjusting the reaction composition to a preset temperature to convert the reaction composition into gel state, and performing a catalytic reaction; (iii) adjusting the temperature of the reaction composition to convert the reaction composition into liquid state, and removing the reaction composition.

In a second aspect, the present invention provides a method for sequencing a target nucleic acid molecule, comprising the following steps:
(1) contacting nucleotides with the target nucleic acid molecule to allow the incorporation of a nucleotide, wherein the nucleotides carry a blocking group;
(2) detecting the nucleotide as incorporated;
(3) removing the blocking group;
wherein, at least one of steps (1) and (2) is performed in gel state.

According to one embodiment of the present invention, step (1) is performed in gel state.

According to another embodiment of the present invention, at least one of steps of step (2) is performed in gel state.

According to another embodiment of the present invention, steps (1) and (2) are performed in gel state.

According to another embodiment of the present invention, steps (1), (2) and (3) are performed in gel state.

As used herein, the expression "performed in gel state" means that the reaction mixture is in gel state. In some embodiments, the method comprises repeating the above steps in sequence to determine the sequence of the target nucleic acid molecule. In some embodiments, the incorporation of the nucleotide described in step (1) results in formation of a phosphodiester bond. In some embodiments, step (3) allows a complementary chain of the target nucleic acid molecule to undergo an extension reaction.

In some embodiments, step (2) comprises step (2a), adding an affinity reagent carrying a fluorescent label to perform an affinity reaction with the product of step (1); and step (2b) detecting the product of step (2a).

In some embodiments, step (2a) is performed in gel state. In some embodiments, step (2a) is performed in gel state, it comprises the following steps: (i) contacting a reaction composition comprising an affinity reagent carrying a fluorescent label with the product of the previous step, wherein the reaction composition further comprises a polymer having reversible thermogel property, and the reaction composition is in liquid state; (ii) adjusting the reaction composition to a preset temperature to convert the reaction composition into gel state and perform an affinity reaction; (iii) adjusting the temperature of the reaction composition to convert the reaction composition into liquid state and removing the reaction composition.

In some embodiments, the affinity reagent is an antibody against a nucleotide A\T\C\G\U carrying a blocking group, and the antibody can specifically recognize the nucleotide and bind with it. The method of using an affinity reagent to recognize an incorporated base is specifically described in the published patent (US10851410B2). In such embodiments, the fluorescent group is not directly labeled on the nucleotide as incorporated, but is labeled on an affinity reagent (e.g., an antibody), and the affinity reagent can specifically bind to the base, sugar, cleavable blocking group, or a combination of these components in the nucleotide as incorporated, so that the type of nucleotide as incorporated can be identified by the affinity reagent.

In a third aspect, the present invention provides a method for sequencing a target nucleic acid molecule, which comprises the following steps:
(1) contacting nucleotides having a label with the target nucleic acid molecule to allow the incorporation of a nucleotide;
(2) detecting the label of the nucleotide as incorporated;
(3) removing the nucleotide as incorporated and the label carried thereon;
(4) contacting unlabeled nucleotides with the target nucleic acid molecule to allow the incorporation of an unlabeled nucleotide.

According to one embodiment of the present invention, step (1) is performed in gel state.

According to another embodiment of the present invention, steps (1) and (4) are performed in gel state.

According to another embodiment of the present invention, steps (1), (3), and (4) are performed in gel state.

In this article, the expression "performed in gel state" means that the reaction mixture is in gel state. In some embodiments, the method comprises repeating the above steps in sequence to determine the sequence of the target nucleic acid molecule.

In some embodiments, the label is a fluorescent label.

In some embodiments, in step (1), the nucleotide is incorporated in accordance with only the principle of base complementary pairing and binds to the 3' end of the complementary chain of the target nucleic acid without forming a phosphodiester bond. A specific DNA polymerase and/or buffer can be used to prevent the formation of phosphodiester bond, and the DNA polymerase basically has no activity to catalyze the formation of phosphodiester bond, and the buffer basically does not contain divalent cations that promote the formation of phosphodiester bond. Such DNA polymerase and/or buffer are well known to those skilled in the art.

In some embodiments, in step (4), the nucleotide is incorporated by a polymerization reaction and forms a phosphodiester bond. Those skilled in the art understand that since the originally nucleotide as incorporated is removed in step (3), the purpose of step (4) is to incorporate the nucleotide that has been removed in step (3), so the unlabeled nucleotide (nucleotide not carrying a label) is incorporated in step (4) to complete the extension reaction of the complementary chain of the target nucleic acid molecule. In some embodiments, the nucleotide incorporated in step (4) is a nucleotide carrying a blocking group to ensure that only one nucleotide can be incorporated in each extension reaction.

In some embodiments, step (3) further comprises removing the blocking group remaining on the 3' end of the target nucleic acid complementary chain. The remaining blocking group refers to a modification carried on the 3' end nucleotide of the complementary chain of the target nucleic acid (i.e., the nucleotide adjacent to the removed nucleotide), which can block the continued incorporation of other nucleotides after the removal of the nucleotide incorporated and the label carried thereon in step (3). The adjacent nucleotide is incorporated by the previous round of step (4). In some embodiments, step (3) will allow the extension reaction of the complementary chain of the target nucleic acid molecule.

In some embodiments, in any of the above aspects, the "performed in gel state" described herein refers to that a reaction composition comprising a polymer having reversible thermogel property is contacted with the surface of a solid phase support on which the target nucleic acid molecule is fixed, a preset temperature is provided to convert the reaction composition into gel state, and other conditions required for reaction are provided to carry out the corresponding sequencing biochemical reaction. After the reaction, the temperature is changed to convert the reaction composition into liquid state, and washing is performed, so that the next step reaction can be performed. The sequencing biochemical reaction described herein may comprise an incorporation reaction (comprising a base extension reaction or a polymerization reaction), a regeneration reaction to remove fluorescent group and/or blocking group, a sequencing primer hybridization, and a reaction to detect the nucleotide incorporated (for example, an affinity reaction and a catalytic reaction in a bioluminescent sequencing method, an affinity reaction of a fluorescently labeled affinity reagent), etc. In certain embodiments, the contact refers to dripping or spraying the liquid reaction composition onto the surface of the solid phase support.

In certain embodiments, in any of the above aspects, the sequencing reaction can be performed in partitions on the same solid phase chip. For example, a sequencing chip is divided into several areas, the first area is used for incorporation reaction, the second area can be used for signal detection, and the third area can be used for elution reaction, etc. Corresponding to the partition sequencing method, the solid phase support is preferably a semiconductor detection chip, the temperature of the solid phase support is preferably controlled in partitions, and the detection signal can be excited fluorescence or bioluminescence. In certain embodiments, the sequencing reaction can be carried out in partitions on the same solid phase chip, for example, a liquid reaction composition is dripped or sprayed onto different areas on the surface of the solid phase support, an incorporation reaction reagent is dripped or sprayed onto the first area to carry out an incorporation reaction, a scanning reagent is dripped or sprayed onto the second area to carry out signal detection, an elution or regeneration reagent (the regeneration reagent is an excision reagent) is dripped or sprayed onto the third area to carry out an elution or regeneration reaction (the regeneration reaction is an excision reaction), etc. Corresponding to the partition sequencing method, the solid phase support is preferably a semiconductor detection chip, and the temperature of the solid phase support is preferably controlled in partitions. The detection signal can be excited fluorescence or bioluminescence. When the detection signal is bioluminescence, the dripped or sprayed scanning reagent is replaced with a modified luciferase or enzyme-catalyzed substrate. The bioluminescence sequencing method and sequencing reagent are described in detail in patent US20220205036A1. In certain embodiments, the sequencing reaction can be carried out in partitions on the same solid phase chip, and the liquid reaction composition is dripped or sprayed onto different areas on the surface of the solid phase support, and then the residual reagents after the reaction on the solid phase surface are removed by an air knife.

### Detection of nucleotide incorporation

It is easy to understand that in any of the above aspects, the nucleotide incorporation is achieved by detecting the label directly or indirectly carried thereby. In the case of using labeled nucleotide, the signal directly or indirectly generated by the label carries the identity information of the nucleotide, and the nucleotide at the position complementarily pairing the nucleotide on the sequenced nucleic acid molecule can be known by the signal. In addition, unlabeled nucleotide can also be used. In the case of using unlabeled nucleotide, an affinity reagent carrying a label (e.g., an antibody) is used to specifically bind to a base, sugar, cleavable blocking group or a combination of these components of the nucleotide as incorporated so as to achieve indirect labeling of the nucleotide as incorporated. The term "label" refers to any detectable molecule that can directly or indirectly generate a detectable signal (including but not limited to optical signal, electrical signal, electromagnetic signal, radioactive signal, etc.).

In certain embodiments, the label is a fluorescent label. Methods for detecting fluorescent labels are well known in the art. For example, it can be achieved by a device that detects the wavelength of fluorescence. Such devices are well known in the art. For example, such a device can be a confocal scanning microscope that scans the surface of a solid phase support with a laser to image the fluorophore directly bound to the nucleic acid molecule being sequenced. In addition, each signal generated can be observed, for example, with a sensitive 2-D detector such as a charge-coupled detector (CCD). Other techniques such as scanning near-field optical microscopy (SNOM) can also be used, and semiconductor detection chips can also be used to convert optical signals into electrical signals for transmission and detection using photodiodes.

In some embodiments, the label is an affinity label. Affinity labels are used in sequencing technologies based on bioluminescent reactions, see, for example, US20220205036A1. The detection principle of bioluminescence comprises that the nucleotide as incorporated is not directly labeled with fluorescent signal, but labeled with an affinity substance such as biotin or digoxigenin. After the polymerization reaction, a pairing member of the above affinity substance with luciferase is added so as to bind the luciferase to the nucleotide as incorporated, and then a substrate of the luciferase is added to generate a light signal for identifying the identity of the nucleotide as incorporated.

In some embodiments, the affinity label is a member of any molecular pair that can specifically bind to each other. The specific binding between the pairing members realizes the connection between the nucleotide and the luciferase. In some embodiments, the label carried by the nucleotide is selected from the group consisting of biotin, digoxigenin, N3G or FITC, and the luciferase carries a pairing member corresponding to the label. For example, if the label carried by the nucleotide is biotin, the luciferase can be a luciferase labeled with streptavidin; if the label carried by the nucleotide is digoxigenin, the luciferase can be a luciferase labeled with a digoxigenin antibody. The source of the luciferase includes but is not limited to firefly, gaussia, Renilla and other organisms.

### Sequencing reaction

It is easy to understand that in any of the above aspects, the nucleotide incorporation described herein refers to incorporation into the complementary chain of the target nucleic acid molecule, which may or may not form a phosphodiester bond. The nucleotides are incorporated successively from the 5' to 3' direction and follow the Watson-Crick base complementary pairing rules. Therefore, the sequence of the target nucleic acid molecule can be inferred by the types of the nucleotides as incorporated detected and in accordance with the Watson-Crick base complementary pairing rules.

It is easy to understand that step (1) in each cycle will only allow one nucleotide to be incorporated into the complementary chain of the target polynucleotide, so the nucleotide to be incorporated has a modification that prevents extension, and the modification prevents the polymerase from continuing to catalyze the incorporation of another nucleotide after incorporating the nucleotide containing the modification into the polynucleotide chain being synthesized.

In certain embodiments, the modification can be an independent blocking group (e.g., a 3' blocking group), and the blocking group is typically located on the ribose ring of the nucleotide to block the interaction with the 3' hydroxyl group. In such embodiments, the labeled nucleotide described in step (1) further comprise an independent blocking group.

In certain embodiments, the modification may also be a label that has a blocking effect itself. Without being bound by theory, such a label may have a size or structure sufficient to block the incorporation of other nucleotides into the polynucleotide chain. The blocking may be caused by steric hindrance, or may be caused by a combination of size, charge and structure. In such embodiments, the label of the labeled nucleotide described in step (1) can be used as a blocking group.

In certain embodiments, the label carried by the labeled nucleotide may be chemically bonded to the nucleotide or may be bonded to the nucleotide in the form of a complex.

It is easy to understand that in any of the above aspects, after the end of one cycle, further incorporation of nucleotides (i.e., continued extension of the complementary chain of the target polynucleotide) will be allowed, so step (3) comprises removing any modification that prevents the continued extension of the complementary chain of the target polynucleotide.

When the incorporation of the nucleotide described in step (1) results in formation of a phosphodiester bond, for example, in the first or second aspect, step (3) comprises removing the modification on the nucleotide as incorporated that prevents extension.

When the nucleotide incorporation described in step (1) does not form a phosphodiester bond, for example, in the third aspect, step (3) comprises directly removing the nucleotide as incorporated. If the nucleotide as incorporated is removed in step (3), it will further comprise step (4), contacting unlabeled nucleotides with the target nucleic acid molecule to allow the incorporation of a unlabeled nucleotide and complete the extension reaction of the complementary chain of the target nucleic acid molecule (i.e., re-incorporating the nucleotide removed in step (3)). The unlabeled nucleotide described in step (4) should have a blocking group to ensure that only one nucleotide is incorporated in this step. In addition, in step (3), the blocking group carried by the nucleotide adjacent to the removed nucleotide (i.e., the unlabeled nucleotide with a blocking group incorporated by step (4) of the previous round of reaction) will also be removed to allow further incorporation of step (4) in this round. Alternatively, after step (4), the blocking group of the nucleotide incorporated in step (4) is removed to allow the next round of sequencing reaction cycle to proceed.

In certain embodiments, when the nucleotide as incorporated results in formation of a phosphodiester bond and the nucleotide as incorporated comprises an independent blocking group and a label, for example, in the first aspect, step (3) further comprises removing the blocking group to allow extension. The removal of the blocking group and the removal of the label can be performed simultaneously or sequentially in any order. The removal of the blocking group and the removal of the label can use the same reagent or different reagents.

In certain embodiments, when the nucleotide as incorporated results in formation of a phosphodiester bond and the nucleotide as incorporated comprises a label that can be used as a blocking group, for example, in the first aspect, when the label is removed in step (3), its blocking effect is also removed at the same time to allow extension.

In certain embodiments, when the nucleotide as incorporated does not form a phosphodiester bond, for example, in the third aspect, when the nucleotide as incorporated is removed in step (3), the label connected thereto is also removed at the same time, and the remaining blocking group is also removed at the same time. It can be understood that under a suitable condition, an elution reagent is used to remove the nucleotide and the label it carries, as well as to remove the remaining blocking group.

In some embodiments, for example in the first aspect, the method of the present invention comprises the following steps: (1) contacting nucleotides with the target nucleic acid molecule to allow the incorporation of a nucleotide and the formation of a phosphodiester bond, wherein the nucleotides carry a blocking group and a label; (2) detecting the label of the nucleotide as incorporated; (3) removing the blocking group and the label from the nucleotide as incorporated to allow extension; wherein at least one of steps (1) and (3) is performed in gel state.

In some other certain embodiments, for example in the first aspect, the method of the present invention comprises the following steps: (1) contacting nucleotides having a label with the target nucleic acid molecule to allow the incorporation of a nucleotide and the formation of a phosphodiester bond, wherein the label can be used as a blocking group; (2) detecting the label of the nucleotide as incorporated; (3) removing the label from the nucleotide as incorporated and removing its blocking effect to allow extension; wherein at least one of steps (1) and (3) is performed in gel state.

In some other certain embodiments, for example in the third aspect, the method of the present invention comprises the following steps: (1) contacting nucleotides having a label with the target nucleic acid molecule to allow the incorporation of a nucleotide but without forming a phosphodiester bond; (2) detecting the label of the nucleotide as incorporated; (3) removing the labeled nucleotide as incorporated under a suitable condition using an elution reagent and removing the remaining blocking group; (4) contacting the target nucleic acid molecule with unlabeled nucleotides to allow the incorporation of a unlabeled nucleotide and the formation of a phosphodiester bond, wherein the unlabeled nucleotides carry a blocking group; wherein at least one of steps (1), (3) and (4) is performed in gel state.

In certain embodiments, in the sequencing reaction of any of the above aspects, under the action of a polymerase, a nucleotide (e.g., nucleotide carrying blocking group and/or label) is incorporated into the complementary chain of the target nucleic acid molecule, and this process is an incorporation reaction. The incorporation reaction may or may not form a phosphodiester bond. When the incorporation reaction results in formation of a phosphodiester bond, it may also be referred to as a polymerization reaction. There are many different polymerases, and it is easy for a person skilled in the art to determine the most suitable polymerase. Preferred enzymes include DNA polymerase I, Klenow fragment, DNA polymerase III, T4 or T7 DNA polymerase, Taq polymerase or vent polymerase. Polymerases modified by engineering methods to have specific properties can also be used. In the sequencing method provided in the present application, the incorporation reaction can be carried out in gel state.

In certain embodiments, in the sequencing reaction of any of the above aspects, when the incorporated labeled nucleotide results in formation of a phosphodiester bond, the reaction of removing the modification (e.g., blocking group and/or label) on the nucleotide as incorporated (e.g., it can be the removal of an independent blocking group and/or label, or it can be the removal of a label with a blocking effect) to allow the next round of sequencing is called an excision reaction. In the sequencing method provided in the present application, the excision reaction can be carried out in gel state.

In certain embodiments, in the sequencing reaction of any of the above aspects, when the incorporated labeled nucleotide does not form a phosphodiester bond, the reaction of removing the nucleotide as incorporated is called an elution reaction. The elution reaction can also comprise removing the remaining blocking group. In the sequencing method provided in the present application, the elution reaction can be carried out in gel state.

In certain embodiments, in the first aspect, steps (1) and (3) are both performed in gel state. In certain embodiments, only step (1) is performed in gel state. In certain embodiments, only step (3) is performed in gel state. In certain embodiments, the affinity reaction in step (2) is also performed in gel state.

In certain embodiments, in the third aspect, steps (1), (3) and (4) are all performed in gel state. In certain embodiments, only step (1) is performed in gel state. In certain embodiments, only step (3) is performed in gel state. In certain embodiments, only steps (1) and (4) are performed in gel state.

In certain embodiments, in the second aspect, steps (1) and (2) are both performed in gel state. In certain embodiments, only step (1) is performed in gel state. In certain embodiments, only step (2) is performed in gel state. In certain embodiments, steps (1) to (3) are all performed in gel state.

In certain embodiments, in the first or third aspect, step (1) is performed in gel state, it comprises the following steps:
(1a) contacting a reaction composition comprising nucleotides having a label with the target nucleic acid molecule, wherein the reaction composition comprises a polymer having reversible thermogel property, and the reaction composition is in liquid state;
(1b) adjusting the reaction composition to a preset temperature to convert the reaction composition into gel state, and performing an incorporation reaction to allow the incorporation of the nucleotide; preferably, the preset temperature allows the incorporation reaction to occur;
(1c) adjusting the temperature of the reaction composition to convert the reaction composition into liquid state.

In some embodiments, the labeled nucleotide further comprises an independent blocking group.

In other embodiments, the label contained in the labeled nucleotide can be used as a blocking group.

In some embodiments, after the reaction composition is converted into liquid state in step (1c), the following steps are further comprised: removing the reaction composition or directly entering the detection reaction of step (2).

In other embodiments, step (1c) is omitted, and step (1b) is followed by the detection reaction of step (2).

In certain embodiments, when the incorporation described in step (1) results in formation of a phosphodiester bond, step (3) is an excision reaction and can be achieved by using a removal reagent. As used herein, "removal reagent" refers to a reagent that can remove a modification on the nucleotide as incorporated. The modification can comprise an independent label and a blocking group, or it can also be a label that can be used as a blocking group. The choice of the removal reagent depends on the modification carried by the nucleotide as incorporated used.

In certain embodiments, when the incorporation described in step (1) does not form a phosphodiester bond, step (3) is an elution reaction and can be achieved by using an elution reagent. In this article, "elution reagent" refers to an elution reagent that can remove the nucleotide as incorporated (and any modification it carries) under appropriate conditions. The elution reagent may also comprise a reagent that can remove the remaining blocking group.

In certain embodiments, in the second aspect, step (1) is performed in gel state, it comprises the following steps:
(1a) contacting a reaction composition comprising nucleotides with the target nucleic acid molecule, wherein the nucleotides carry a blocking group, the reaction composition comprises a polymer having reversible thermogel property, and the reaction composition is in liquid state;
(1b) adjusting the reaction composition to a preset temperature to convert the reaction composition into gel state, and performing an incorporation reaction to allow the incorporation of the nucleotides; preferably, the preset temperature allows the incorporation reaction to occur;
(1c) adjusting the temperature of the reaction composition to convert the reaction composition into liquid state.

In certain embodiments, in any of the above aspects, step (3) is performed in gel state, it comprises the following steps:
(3a) contacting a reaction composition comprising a removal agent or an elution agent with the product of the previous step, wherein the reaction composition further comprises a polymer having reversible thermogel property, and the reaction composition is in liquid state;
(3b) adjusting the reaction composition to a preset temperature to convert the reaction composition into gel state, and performing an excision reaction or an elution reaction to allow the removal of the label or blocking group on the nucleotide or the removal of the nucleotide as incorporated; preferably, the preset temperature allows the occurrence of the excision reaction or the elution reaction;
(3c) adjusting the temperature of the reaction composition to convert the reaction composition into liquid state, and removing the reaction composition.

In some embodiments, in the first aspect, step (3) is an excision reaction, and when the labeled nucleotide in step (1) further comprises an independent blocking group, step (3) comprises using a removal agent to remove the blocking group and the label from the nucleotide as incorporated. In such embodiments, step (3) is performed in gel state, it comprises the following steps:
(3a) contacting a reaction composition comprising a removal agent with the product of the previous step, wherein the reaction composition further comprises a polymer having reversible thermogel property, and the reaction composition is in liquid state; the removal agent comprises an agent capable of removing the label and the blocking group;
(3b) adjusting the reaction composition to a preset temperature to convert the reaction composition into gel state, and performing an excision reaction to allow the removal of the blocking group and the label on the nucleotide; preferably, the preset temperature allows the occurrence of the excision reaction;
(3c) adjusting the temperature of the reaction composition to convert the reaction composition into liquid state, and removing the reaction composition.

In other embodiments, in the first aspect, step (3) is an excision reaction, and when the label contained in the nucleotide carrying the label in step (1) can be used as a blocking group, step (3) comprises using a removal agent to remove the label from the nucleotide as incorporated and simultaneously remove its blocking effect. In such embodiments, step (3) is performed in gel state, it comprises the following steps:
(3a) contacting a reaction composition comprising a removal agent with the product of the previous step, wherein the reaction composition further comprises a polymer having reversible thermogel property, and the reaction composition is in liquid state; the removal agent comprises an agent capable of removing the label;
(3b) adjusting the reaction composition to a preset temperature to convert the reaction composition into gel state, and performing an excision reaction to allow the removal of the label on the nucleotide, the removal of the label will simultaneously remove its blocking effect; preferably, the preset temperature allows the occurrence of the excision reaction;
(3c) adjusting the temperature of the reaction composition to convert the reaction composition into liquid state, and removing the reaction composition.

In other embodiments, in the second aspect, step (3) is an excision reaction, which comprises using a removal agent to remove the blocking group from the nucleotide as incorporated. In such embodiments, step (3) is performed in gel state, it comprises the following steps:
(3a) contacting a reaction composition comprising a removal reagent with the product of the previous step, wherein the reaction composition further comprises a polymer having reversible thermogel property, the reaction composition is in liquid state; the removal reagent comprises a reagent capable of removing the blocking group;
(3b) adjusting the reaction composition to a preset temperature to convert the reaction composition into gel state, and performing an excision reaction to allow the removal of the blocking group on the nucleotide; preferably, the preset temperature allows the occurrence of the excision reaction;
(3c) adjusting the temperature of the reaction composition to convert the reaction composition into liquid state, and removing the reaction composition.

In other embodiments, in the third aspect, step (3) is an elution reaction, which comprises using an elution reagent to remove the nucleotide as incorporated and the remaining blocking group. In such embodiments, step (3) is performed in gel state, it comprises the following steps:
(3a) contacting a reaction composition comprising an elution reagent with the product of the previous step, wherein the reaction composition further comprises a polymer having reversible thermogel property, the reaction composition is in liquid state; the elution reagent comprises a reagent capable of removing the nucleotide as incorporated (and any modification it carries) and the remaining blocking group, wherein the remaining blocking group may be a modification carried by the 3' terminal nucleotide of the complementary chain of the target nucleic acid, which blocks the continued incorporation of other nucleotides;
(3b) adjusting the reaction composition to a preset temperature to convert the reaction composition into gel state, and performing an elution reaction to allow the removal of the nucleotide as incorporated and the removal of the remaining blocking group; preferably, the preset temperature allows the elution reaction to occur;
(3c) adjusting the temperature of the reaction composition to convert the reaction composition into liquid state, and removing the reaction composition.

In other embodiments, step (3) is carried out in solution state, it comprises: removing the incorporated labeled nucleotide and the remaining blocking group under appropriate conditions using the elution reagent.

In certain embodiments, in the third aspect, step (4) is performed in gel state, it comprises the following steps:
(4a) contacting a reaction composition comprising unlabeled nucleotides with the target nucleic acid molecule, wherein the reaction composition comprises a polymer having reversible thermogel property, and the reaction composition is in liquid state; preferably, the unlabeled nucleotides have a blocking group to ensure that only one nucleotide can be incorporated into the 3' end of the complementary chain of the target nucleic acid in each round of extension reaction;
(4b) adjusting the reaction composition to a preset temperature to convert the reaction composition into gel state, and performing a polymerization reaction to allow the incorporation of nucleotide;
(4c) adjusting the temperature of the reaction composition to convert the reaction composition into liquid state, and optionally removing the reaction composition.

In certain embodiments, in the first or third aspect, before, during or after any step before step (3) (for example, after step (1) and before step (2)), the method further comprises: a step of contacting unlabeled nucleotides with the target nucleic acid molecule, wherein the unlabeled nucleotides carry a blocking group; the step is optionally performed in gel state. In certain embodiments, the incorporation of the nucleotide in this step results in formation of a phosphodiester bond.

In certain embodiments, in the first or third aspect, after step (1) and before step (2), the method comprises the following steps:
(i) contacting a reaction composition comprising unlabeled nucleotides with the target nucleic acid molecule, wherein the unlabeled nucleotides carry a blocking group, the reaction composition comprises a polymer having reversible thermogel property, and the reaction composition is in liquid state;
(ii) adjusting the reaction composition to a preset temperature to convert the reaction composition into gel state, and performing an incorporation reaction to allow the incorporation of nucleotide; preferably, the preset temperature allows the incorporation reaction to occur;
(iii) adjusting the temperature of the reaction composition to convert the reaction composition into liquid state, and removing the reaction composition.

In certain embodiments, in any of the above aspects, before step (1), the method further comprises: a step of contacting a sequencing primer with the target nucleic acid molecule to allow hybridization; the step is optionally performed in gel state.

In certain embodiments, in any of the above aspects, the method comprises the following steps before step (1):
(i) contacting a reaction composition comprising a sequencing primer with the target nucleic acid molecule, wherein the reaction composition comprises a polymer having reversible thermogel property, and the reaction composition is in liquid state;
(ii) adjusting the reaction composition to a preset temperature to convert the reaction composition into gel state so that the sequencing primer hybridizes to the target nucleic acid molecule; preferably, the preset temperature allows the primer hybridization to occur;
(iii) adjusting the temperature of the reaction composition to convert the reaction composition into liquid state, and removing the reaction composition.

In certain embodiments, in any of the above aspects, the method is a Flowcell sequencing method. The term "Flowcell sequencing" refers to any sequencing method based on a flow cell having a fixed nucleic acid molecule to be detected. Typical Flowcell sequencing method includes, for example, Illumina's SBS sequencing technology. Taking Illumina's HiSeq 2500 platform as an example, the platform uses a flow cell to pump a sequencing reaction reagent from a refrigeration device into the flow cell at a specific position at a specific time point, which flows through a sequencing chip under the control of a set of complex fluid control equipment, and the assistance of a fine temperature control device to ensure that the biochemical reaction can be fully carried out in the shortest possible time.

In certain embodiments, in any of the above aspects, the target nucleic acid molecule is fixed on the surface of the flow cell.

In certain embodiments, in any of the above aspects, the method comprises introducing one or more sequencing reagents required for the sequencing reaction into a flow cell fixed with the target nucleic acid molecule to complete the corresponding sequencing biochemical reaction.

In certain embodiments, in any of the above aspects, the method is an open sequencing method. The term "open sequencing" refers to a sequencing method based on a dipping reaction scheme or a contact reaction scheme, see, for example, the method described in WO2019023951A1. Different from the traditional Flowcell sequencing, in which one or more sequencing reagents are introduced into a flow cell having a target nucleic acid molecule fixed thereon, in the open sequencing, one or more sequencing reagents required for the sequencing reaction are placed in separate reaction containers, and a solid phase support with a nucleic acid molecule to be tested is moved to contact with the one or more sequencing reagents placed in the separate reaction containers, or dipping the solid phase support in the one or more sequencing reagents to complete the corresponding sequencing biochemical reaction. In the open sequencing, the solid phase support having the target nucleic acid molecule fixed thereon can be called an open sequencing slide.

In some embodiments, in any of the above aspects, the target nucleic acid molecule is fixed on the surface of the open sequencing slide.

In some embodiments, in any of the above aspects, the method comprises allowing an open sequencing slide having a target nucleic acid molecule fixed thereon to contact with or dip into one or more sequencing reagents required for the sequencing reaction, respectively, wherein the one or more sequencing reagents are placed in separate reaction containers.

In some embodiments, in any of the above aspects, the method provided by the present invention is particularly suitable for an open sequencing platform. The method of the present invention has the advantages of open schemes, such as reduced cost, simple fluids, and support for ultra-large chips, and solves some problems of open schemes, such as chip drying, sample damage, and the inability to automate some special processes (e.g., sample loading).

### Thermogel polymer

The polymer having reversible thermogel property described herein refers to a polymer that responds to temperature change by changing from liquid to gel when the temperature rises.

In some embodiments, the concentration of the polymer is about 0.5~30% (w/w), such as about 0.5~5% (w/w), 0.5~2% (w/w), about 5~10% (w/w), about 10~15% (w/w), about 15~20% (w/w), about 20~25% (w/w), about 25~30% (w/w).

In some embodiments, the concentration of the polymer is about 10~30% (w/w), such as about 15~25% (w/w), about 18~22% (w/w), such as about 20% (w/w). In some embodiments, the polymer is selected from Pluronic block polymers.

In some embodiments, the concentration of the polymer is about 0.5~10% (w/w), such as about 0.5~5% (w/w), about 0.5~2% (w/w), about 1~2% (w/w), such as about 1% (w/w). In some embodiments, the polymer is selected from methylcellulose or hydroxypropyl methylcellulose.

In some embodiments, the polymer is a block copolymer, a graft copolymer, or a homopolymer. In certain embodiments, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxypolyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA), and the like. Pluronic block polymers can be selected from Pluronic F38, P65, P68LF, P75, F77, P84, P85, F87, F88, F98, P103, P104, P105, F108, P123, F123, F127, 10R8, 17R8, 25R5, 25R8, for example, Pluronic F127. Methylcellulose can be selected from, Methocel A, Methocel A4C, Methocel A15C, Methocel A15 LV, Methocel A4M, for example, Methocel A15C or Methocel A15 LV. Hydroxypropyl methylcellulose can be selected from, Methocel E5LV, Methocel E3LV, Methocel E6LV, Methocel E15LV, Methocel E50LV and Methocel K3LV.

In some embodiments, the polymer has a gelling concentration of about 0.5% to 25% (w/w). In some embodiments, the polymer has a gelling concentration of about 0.5% to 5% (w/w), about 5% to 10% (w/w), about 10% to 15% (w/w), about 15% to 20% (w/w) or a gelling concentration of about 20% to 25% (w/w).

In some embodiments, the polymer has a gelling temperature of about 10~65°C. In some embodiments, the polymer has a gelling temperature of about 10~15°C, about 15~20°C, about 20~25°C, about 25~30°C, about 30~35°C, about 35~40°C, about 40~45°C, about 45~50°C, about 50~55°C, about 55~60°C or about 60~65°C.

In some embodiments, the temperature at which the polymer having reversible thermogel property is converted from gel state to liquid state is about 0~30°C.

In some embodiments, the temperature at which the polymer having reversible thermogel property is converted from gel state to liquid state is about 0~10°C, such as about 2~8°C, such as about 4°C. In some embodiments, the polymer is selected from Pluronic block polymers. In some embodiments, the concentration of the polymer is about 10~30% (w/w), such as about 15~25% (w/w), about 18~22% (w/w), such as about 20% (w/w).

In some embodiments, the temperature at which the polymer having reversible thermogel property is converted from gel state to liquid state is about 20~30°C, such as about 20~25°C, such as about 20°C or about 25°C. In some embodiments, the polymer is selected from methylcellulose or hydroxypropyl methylcellulose. In some embodiments, the concentration of the polymer is about 0.5~10% (w/w), such as about 0.5~5% (w/w), about 0.5~2% (w/w), about 1~2% (w/w), such as about 1% (w/w).

In certain embodiments, in any step of the method of the present application, the preset temperature refers to a temperature that allows a reaction composition comprising a polymer having reversible thermogel property to be converted from liquid state to gel and allows the desired reaction to occur. The desired reaction can be any one of sequencing biochemical reactions, such as an incorporation reaction (including a base extension reaction or a polymerization reaction), a regeneration reaction to remove a fluorescent group and/or a blocking group, a sequencing primer hybridization, and a reaction to detect the incorporation of nucleotide (for example, an affinity reaction and a catalytic reaction in a bioluminescent sequencing method, an affinity reaction of a fluorescently labeled affinity reagent), etc. Some exemplary preset temperatures are provided below, but those skilled in the art are fully capable of adjusting the preset temperature according to actual needs. For example, for an incorporation reaction, if the suitable reaction temperature of polymerase is 37°C, the corresponding preset temperature can be set to 37°C. For example, for primer hybridization, if the suitable temperature is 60°C, the corresponding preset temperature can be set to 60°C.

For example, when the reaction composition is used for an incorporation reaction, the preset temperature should allow the incorporation reaction to occur. In some embodiments, the preset temperature for converting a reaction composition comprising nucleotides (e.g., labeled nucleotides, unlabeled nucleotides, or unlabeled nucleotides with a blocking group) into gel state is about 50~60°C, such as about 55~60°C, such as about 55°C.

For example, when the reaction composition is used for an excision reaction, the preset temperature should allow the removal of a blocking group and/or a fluorescent group incorporated into the nucleotide. In some embodiments, the preset temperature for converting a reaction composition comprising a removal reagent into gel state is about 50~60°C, such as about 55~60°C, such as about 55°C.

For example, when the reaction composition is used for an elution reaction, the preset temperature should allow the removal of the nucleotide as incorporated and the removal of the remaining blocking group. In some embodiments, the preset temperature for converting a reaction composition comprising an elution reagent into gel state is about 50~60°C, such as about 55~60°C, such as about 55°C.

For example, when the reaction composition is used for a primer hybridization (e.g., when a sequencing primer hybridize to an MDA chain generated by MDA amplification), the preset temperature should allow the primer hybridization to occur. In certain embodiments, the preset temperature for converting a reaction composition comprising a sequencing primer into gel state is about 35~45°C, such as about 37~45°C, about 37~42°C, such as about 37°C.

For example, when the reaction composition is used for an affinity reaction and a catalytic reaction in a bioluminescent sequencing method, the preset temperature should allow the affinity reaction and catalytic reaction to occur, and is, such as about 30~40°C, such as about 30~37°C, about 32~37°C, about 35~37°C, such as about 35°C.

For example, when the reaction composition is used for an affinity reaction of a fluorescently labeled affinity reagent, the preset temperature should allow the affinity reaction to occur, and is, such as about 30~40°C, such as about 30~37°C, about 32~37°C, about 35~37°C, such as about 35°C.

### Reaction composition

In any reaction step of the method of the present application, the reaction composition comprising a polymer having reversible thermogel property comprises any necessary component that allow the corresponding reaction to occur.

In certain embodiments, when the reaction composition is used for an incorporation reaction, the necessary component may be an enzyme (e.g., a polymerase, such as a DNA polymerase), a primer, a buffer, or any combination thereof. In certain embodiments, the reaction composition for the incorporation reaction comprises nucleotides to be incorporated and an enzyme (e.g., a polymerase, such as a DNA polymerase), and each of the nucleotides to be incorporated may be a nucleotide carrying a blocking group and a label, an unlabeled nucleotide carrying a blocking group, an unlabeled nucleotide, or any combination thereof.

In the text, the term "nucleotide" is expected to include natural nucleotides, non-natural nucleotides, ribonucleotides, deoxyribonucleotides, dideoxyribonucleotides and analogs thereof. For example, the term generally refers to a nucleoside moiety (regardless of ribose, deoxyribose or analog thereof) containing a base portion and optionally attached to one or more phosphate moieties herein. The term can be used to refer to a monomer unit present in a polynucleotide, for example, to identify a subunit present in a DNA or RNA chain. The term can also be used to refer to a monomer molecule that is not necessarily present in a polynucleotide, for example, a molecule that can be incorporated into a polynucleotide in a template-dependent manner by a polymerase. Exemplary nucleotides include, but are not limited to, monophosphate ribonucleotides (sometimes referred to as ribonucleoside monophosphates), diphosphate ribonucleotides (sometimes referred to as ribonucleoside diphosphates), triphosphate ribonucleotides (sometimes referred to as ribonucleoside triphosphates), monophosphate deoxynucleotides (sometimes referred to as deoxynucleoside monophosphates), diphosphate deoxynucleotides (sometimes referred to as deoxynucleoside diphosphates) and triphosphate deoxynucleotides (sometimes referred to as deoxynucleoside triphosphates). For clarity, when it is desired to distinguish RNA components from DNA components, the term "ribonucleotide" may be used to designate RNA nucleotides, such as ribouridine triphosphate, riboguanosine triphosphate, ribocytidine triphosphate, or riboadenosine triphosphate; and the term "deoxynucleotide" is used to designate RNA nucleotides, such as deoxythymidine triphosphate, deoxyguanosine triphosphate, deoxycytidine triphosphate, or deoxyadenosine triphosphate.

In certain embodiments, each of the nucleotides to be incorporated is a dNTP or analog thereof. In certain embodiments, each of the nucleotides to be incorporated is selected from deoxyadenosine triphosphate (dATP) or analog thereof, deoxythymidine triphosphate (dTTP) or analog thereof, deoxycytidine triphosphate (dCTP) or analog thereof, deoxyguanosine triphosphate (dGTP) or analog thereof. In certain embodiments, the analog means a synthetic nucleotide having a modified base portion, a modified phosphodiester linkage, and/or a modified sugar portion.

In certain embodiments, each of the nucleotides to be incorporated is a labeled nucleotide. In certain embodiments, the label is a fluorescent group.

In some embodiments, each of the nucleotides to be incorporated is a nucleotide carrying an independent blocking group and a label.

The blocking group described herein can prevent further incorporation of nucleotide into the complementary strand of the target nucleic acid molecule. A skilled person will understand how to attach a suitable blocking group to the ribose ring of the nucleotide to block the interaction with the 3' hydroxyl group. The blocking group can be attached directly at the 3' position or can be attached at the 2' position (the blocking group has a sufficient size or charge to block the interaction at the 3' position). Alternatively, the blocking group can be attached at both the 3' and 2' positions and can be cleaved to expose the 3' hydroxyl group. Suitable blocking groups will be obvious to those skilled in the art.

In other embodiments, each of the nucleotides to be incorporated is a nucleotide carrying only a label, and the label can be used as a blocking group. Without being bound by theory, such a label can have a size or structure sufficient to block the incorporation of other nucleotides into the polynucleotide chain. The blocking may be due to steric hindrance, or may be due to a combination of size, charge and structure. Such labels will be obvious to those skilled in the art.

In certain embodiments, the blocking group and/or label can be connected to the nucleotide via a cleavable linker. In certain embodiments, the linker comprises one or more cleavable groups. In certain embodiments, the linker is selected from the group consisting of: disulfide linker, acid-labile linker (e.g., dialkoxybenzyl linker, Sieber linker, indole linker, tert-butyl Sieber linker), electrophilic cleavable linker, nucleophilic cleavable linker, photocleavable linker, or a combination thereof.

The types of disulfide linkers, acid-labile linkers (e.g., dialkoxybenzyl linker, Sieber linker, indole linker, tert-butyl Sieber linker), electrophilic cleavable linkers, nucleophilic cleavable linkers, photocleavable linkers are obvious to those skilled in the art. For example, electrophilic cleavable linkers are typically cleaved by protons and include acid-sensitive cleavage. Suitable electrophilic cleavable linkers include modified benzyl systems such as trityl, p-alkoxybenzyl ester, and p-alkoxybenzyl amide. Other suitable electrophilic cleavable linkers include tert-butyloxycarbonyl (Boc) group and acetal system. In addition, it is also possible to consider using sulfur-philic metals such as nickel, silver or mercury in the cleavage of thioacetal or other sulfur-containing protecting groups to prepare suitable electrophilic cleavable linker molecules. Nucleophilic cleavage is a recognized method for preparing linker molecules. For example, groups that are unstable in water such as esters (i.e., can be simply cleaved at alkaline pH) and groups that are unstable to non-aqueous nucleophiles can be used. It is preferred for the photocleavable linker that the light required for activating cleavage does not affect other components of the modified nucleotide. For example, if a fluorescent group is used as a label, it is preferred that the light required for absorption and the light for cleavage of linker molecule are different in wavelength.

In some embodiments, each of the nucleotides to be incorporated is a nucleotide that carries a blocking group and is not labeled. The incorporation of such nucleotide will help to supplementally incorporate the part of incomplete reaction, ensure the synthesis efficiency, and reduce the risk of incomplete reaction; and because such nucleotide is not labeled, the signal interference caused by unclean excision of the label or unclean elution of the excised label is also reduced, the efficiency of excision is improved, and the sequencing error rate is reduced.

In some embodiments, each of the nucleotides to be incorporated is an unlabeled nucleotide. When the incorporated labeled nucleotide does not form a phosphodiester bond, the step of removing the label will simultaneously remove the nucleotide as incorporated. In such embodiments, an unlabeled nucleotide needs to be added again to allow the next round of sequencing reaction to proceed. In certain embodiments, the unlabeled nucleotide refers to a nucleotide that does not carry a label but has a blocking group.

In certain embodiments, each of the nucleotides to be incorporated can form a phosphodiester bond.

In certain embodiments, each of the nucleotides to be incorporated cannot form a phosphodiester bond.

### Solid phase support

In certain embodiments, the target nucleic acid molecule is fixed to a solid phase support.

As used herein, the term "fixed" when used in reference to nucleic acid means that it is directly or indirectly attached to a solid phase support via a covalent or non-covalent bond. In certain embodiments, the nucleic acid is covalently attached to a solid phase support. In certain embodiments, fixing a nucleic acid on a solid phase support may comprise fixing an oligonucleotide to be used as a capture primer or an amplification primer on the solid phase support so that the 3' end is available for enzymatic extension and at least a portion of the primer sequence is hybridizable to a complementary nucleic acid sequence; then hybridizing a nucleic acid to be fixed to the oligonucleotide, in which case the fixed oligonucleotide or polynucleotide may be in a 3' to 5' direction. In certain embodiments, fixing a nucleic acid on a solid phase support may comprise binding a nucleic acid binding protein to a solid phase support by amino modification, and capturing a nucleic acid molecule by the nucleic acid binding protein. Non-limiting examples of attachment of nucleic acids to solid phase supports include nucleic acid hybridization, biotin-streptavidin binding, thiol binding, photoactivated binding, covalent binding, antibody-antigen, physical confinement via hydrogel or other porous polymers, and the like.

As used therein, the support may be made of a variety of suitable materials. Such materials include, for example, inorganic substances, natural polymers, synthetic polymers, and any combination thereof. Specific examples include, but are not limited to, cellulose, cellulose derivatives (e.g., nitrocellulose), acrylic resins, glass, silica gel, silicon dioxide, polystyrene, gelatin, polyvinyl pyrrolidone, copolymers of vinyl and acrylamide, polystyrene cross-linked with divinylbenzene, etc. (see, for example, Merrifield Biochemistry 1964, 3, 1385~1390), polyacrylamide, latex, dextran, rubber, silicon, plastic, natural sponge, metal plastic, cross-linked dextran (e.g., Sephadex^{™}), agarose gel (Sepharose^{™}), and other supports known to those skilled in the art.

In certain embodiments, the support for fixing nucleic acid molecule to be sequenced can be a solid phase support comprising an inert substrate or matrix (e.g., glass slide, polymer beads, etc.), the inert substrate or matrix has been functionalized, for example, by applying an intermediate material containing an active group, and the active group allows covalent attachment of a biological molecule such as polynucleotide. In certain embodiments, the support is a glass or silicon wafer with a layer of avidin, amino, acrylamide silane or aldehyde chemical groups modified on its surface.

Herein, the solid phase support is not limited in size, shape and configuration. In some embodiments, the solid phase support is a planar structure, such as a slide, a chip, a microchip and/or an array. The surface of such support may be in the form of a planar layer.

In certain embodiments, the support for fixing nucleic acid molecule to be sequenced is a chip, such as a high-throughput sequencing chip.

### Multiple copies of target nucleic acid molecule

In order to enhance the detection signal of the label, a person skilled in the art may choose to form multiple copies of the target nucleic acid molecule to be detected, and the number of copies is not limited. In such embodiments, multiple copies of the target nucleic acid molecule are connected in the form of nucleic acid clusters to the solid phase support. In certain embodiments, the nucleic acid clusters are prepared by a solid phase amplification method of bridge amplification. In certain embodiments, the nucleic acid clusters are prepared by rolling circle amplification to form a DNA nanoball (DNB).

In certain embodiments, the target nucleic acid molecule is present in a nucleic acid array. In certain embodiments, each site on the array may comprise multiple copies of single target nucleic acid molecule.

In certain embodiments, the target nucleic acid molecule may be a DNB formed by multiple copies of single target nucleic acid molecule.

DNA nanoball (DNB) is a concatemer containing multiple copies of target nucleic acid molecule. These nucleic acid copies are typically arranged one after another in a continuous linear chain of nucleotides, and this tandem repeat structure together with the single-stranded nature of DNA causes the configuration of nanoball folding. In general, the multiple copies of target nucleic acid molecule in the DNB each contain a linker sequence with a known sequence to facilitate amplification or sequencing thereof. The linker sequences of each target nucleic acid molecule are usually the same, but may also be different. The DNB can be produced using, for example, rolling circle replication (RCA).

The DNB can be loaded on the surface of the solid phase support as described herein. The DNB can be attached to the surface of the solid phase support by any suitable method, and non-limiting examples of such method include nucleic acid hybridization, biotin-streptavidin binding, thiol binding, photoactivated binding, covalent binding, antibody-antigen, physical confinement via hydrogel or other porous polymers, etc., or a combination thereof. In some embodiments, the surface of the solid phase support may carry a reactive functional group, and the reactive functional group reacts with a complementary functional group on the polynucleotide molecule to form a covalent bond, for example, in the same manner as the techniques used to attach cDNA to a microarray. The DNB can also be effectively attached to a hydrophobic surface, such as a clean glass surface with low concentration of various reactive functional groups (e.g., -OH group). In other embodiments, the DNB can be adsorbed onto the surface. In such embodiment, the polynucleotide is fixed by a non-specific interaction with the surface, or by non-covalent interaction such as hydrogen bond, van der Waals force, etc.

### Kit

In another aspect, the present invention provides a kit, which comprises:
(a) a composition comprising labeled nucleotides and a polymer having reversible thermogel property, the composition optionally further comprising an enzyme (e.g., a polymerase, such as a DNA polymerase), a primer and/or a buffer; and/or,
(b) a composition comprising a removal agent and a polymer having reversible thermogel property, the composition optionally comprising a buffer.

In some embodiments, each of the labeled nucleotides in (a) further comprises an independent blocking group and is capable of forming a phosphodiester bond. In such embodiments, the removal reagent in (b) comprises a reagent capable of removing the label and a reagent capable of removing the blocking group, and the two reagents may be the same or different.

In other embodiments, the label contained in the labeled nucleotides in (a) can be used as a blocking group and is capable of forming a phosphodiester bond. In such embodiments, the removal reagent in (b) comprises a reagent capable of removing the label, and it is understood by those skilled in the art that the removal of the label will simultaneously remove its blocking effect.

In certain embodiments, the kit further comprises: (c) a composition comprising unlabeled nucleotides and a polymer having reversible thermogel property, wherein the unlabeled nucleotides carry a blocking group, the composition optionally further comprising an enzyme (e.g., a polymerase, such as a DNA polymerase), a primer and/or a buffer. In certain embodiments, the unlabeled nucleotides in (c) is capable of forming a phosphodiester bond.

In certain embodiments, the kit further comprises: (d) a composition comprising a sequencing primer and a polymer having reversible thermogel property, the composition optionally comprising a buffer.

In some embodiments, the polymers having reversible thermogel properties described in any one of (a) to (d) may be the same or different.

In some embodiments, the kit comprises: (a) and (b); (a) and (c); (b) and (c); (a) and (d); (b) and (d); (a), (b) and (c); (a), (b) and (d); or (a), (b), (c) and (d). In some embodiments, the kit is used for sequencing, such as the sequencing method described herein. In some embodiments, the kit also comprises other reagents for sequencing. In some embodiments, when the kit of any of the foregoing items is used for bioluminescent sequencing technology, the kit may further comprise: (e) a composition comprising a luciferase carrying an affinity label and a polymer having reversible thermogel property, and/or (f) a composition comprising a specific substrate and a polymer having reversible thermogel property.

In some embodiments, the temperature at which any composition contained in the kit is converted from gel state to liquid state is about 0~30°C. In some embodiments, the temperature at which the composition is converted from gel state to liquid state is about 0~10°C, such as about 2~8°C, such as about 4°C. In some embodiments, the temperature at which the composition is converted from gel state to liquid state is about 20~30°C, such as about 20~25°C, such as about 20°C or about 25°C.

In some embodiments, the preset temperature at which the composition described in (a) is converted into gel state is about 50~60°C, such as about 55~60°C, such as about 55°C. In some embodiments, the preset temperature at which the composition described in (b) is converted into gel state is about 50~60°C, such as about 55~60°C, such as about 55°C. In some embodiments, the preset temperature at which the composition described in (c) is converted into gel state is about 50~60°C, such as about 55~60°C, such as about 55°C. In some embodiments, the preset temperature at which the composition described in (d) is converted into gel state is about 35~45°C, such as about 37~45°C, about 37~42°C, such as about 37°C. In some embodiments, the preset temperature at which the composition described in (e) is converted into gel state is about 30~40°C, such as about 30~37°C, about 32~37°C, about 35~37°C, such as about 35°C. In some embodiments, the preset temperature at which the composition described in (f) is converted into gel state is about 30~40°C, such as about 30~37°C, about 32~37°C, about 35~37°C, such as about 35°C.

In some embodiments, the kit described in any of the above embodiments may further comprise a washing solution.

On the other hand, the present invention also provides a kit, which comprises:
(1) a composition comprising nucleotides and a polymer having reversible thermogel property, wherein the nucleotides carry a blocking group, the composition optionally further comprising an enzyme (e.g., a polymerase, such as a DNA polymerase), a primer and/or a buffer; and/or,
(2) a composition comprising an affinity reagent carrying a fluorescent label (e.g., an antibody that recognizes nucleotide) and a polymer having reversible thermogel property, the composition optionally comprising a buffer.

In certain embodiments, in (1), the nucleotides are capable of forming a phosphodiester bond.

In certain embodiments, in (2), the affinity reagent is an antibody against any nucleotide A\T\C\G\U carrying a blocking group, and the antibody is capable of specifically recognizing the nucleotide and undergoing affinity reaction therewith.

In certain embodiments, the kit further comprises: (3) a composition comprising a removal reagent and a polymer having reversible thermogel property, the composition optionally comprising a buffer. The removal reagent comprises a reagent capable of removing the blocking group.

In some embodiments, the kit further comprises: (4) a composition comprising a sequencing primer and a polymer having reversible thermogel property, the composition optionally comprising a buffer.

In some embodiments, the kit comprises: (1) and (2); (1) and (3); (1) and (4); (2) and (3); (2) and (4); (1), (2) and (3); (1), (2) and (4); or (1), (2), (3) and (4).

In some embodiments, the temperature at which any composition contained in the kit is converted from gel state to liquid state is about 0 to 30°C. In some embodiments, the temperature at which the composition is converted from gel state to liquid state is about 0~10°C, such as about 2~8°C, such as about 4°C. In some embodiments, the temperature at which the composition is converted from a gel state to a liquid state is about 20~30°C, such as about 20~25°C, such as about 20°C or about 25°C.

In some embodiments, the preset temperature at which the composition of (1) is converted into gel state is about 50~60°C, such as about 55~60°C, such as about 55°C. In some embodiments, the preset temperature at which the composition of (2) is converted into gel state is about 30~40°C, such as about 30~37°C, about 32~37°C, about 35~37°C, such as about 35°C. In some embodiments, the preset temperature at which the composition of (3) is converted into gel state is about 50~60°C, such as about 55~60°C, such as about 55°C. In some embodiments, the preset temperature at which the composition of (4) is converted into gel state is about 35~45°C, such as about 37~45°C, about 37~42°C, such as about 37°C.

On the other hand, the present invention also provides a kit, which comprises:
(i) a composition comprising labeled nucleotides and a polymer having reversible thermogel property, the composition optionally further comprising an enzyme (e.g., a polymerase, such as a DNA polymerase), a primer and/or a buffer;
(ii) a composition comprising an elution reagent and a polymer having reversible thermogel property, the composition optionally comprising a buffer; and/or,
(iii) a composition comprising unlabeled nucleotides and a polymer having reversible thermogel property, the composition optionally further comprising an enzyme (e.g., a polymerase, such as a DNA polymerase), a primer and/or a buffer.

In some embodiments, each of the labeled nucleotides in (i) further comprises an independent blocking group and does not form a phosphodiester bond. In other embodiments, the label contained in the labeled nucleotides in (i) can be used as a blocking group and does not form a phosphodiester bond.

In some embodiments, each of the unlabeled nucleotides in (iii) is capable of forming a phosphodiester bond. In some embodiments, the unlabeled nucleotides in (iii) carry a blocking group.

In some embodiments, the kit further comprises: (iv) a composition comprising unlabeled nucleotides and a polymer having reversible thermogel property, wherein the unlabeled nucleotides carry a blocking group, the composition optionally further comprising an enzyme (e.g., a polymerase, such as a DNA polymerase), a primer and/or a buffer. In some embodiments, (iv) the unlabeled nucleotides are capable of forming a phosphodiester bond.

In some embodiments, the kit further comprises: (v) a composition comprising a sequencing primer and a polymer having reversible thermogel property, the composition optionally comprising a buffer.

In some embodiments, the polymers having reversible thermogel properties described in any one of (i) to (v) may be the same or different.

In some embodiments, the kit comprises: (i) and optionally (iv) and/or (v); (ii) and optionally (iv) and/or (v); (iii) and optionally (iv) and/or (v); (i) and (ii) and optionally (iv) and/or (v); (i) and (iii) and optionally (iv) and/or (v); (ii) and (iii) and optionally (iv) and/or (v); (i), (ii) and (ii) and optionally (iv) and/or (v).

In some embodiments, the kit is used for sequencing, such as the sequencing method described herein. In some embodiments, the kit further comprises other reagents for sequencing. In some embodiments, when any of the foregoing kits is used for bioluminescent sequencing technology, the kit may further comprise: a composition comprising a luciferase carrying an affinity label and a polymer having reversible thermogel property, and/or a composition comprising a specific substrate and a polymer having reversible thermogel property.

In some embodiments, the temperature at which any composition contained in the kit is converted from gel state to liquid state is about 0~30°C. In some embodiments, the temperature at which the composition is converted from gel state to liquid state is about 0~10°C, such as about 2~8°C, such as about 4°C. In some embodiments, the temperature at which the composition is converted from gel state to liquid state is about 20~30°C, such as about 20~25°C, such as about 20°C or about 25°C.

In some embodiments, the preset temperature at which the composition described in (i) is converted into gel state is about 50~60°C, such as about 55~60°C, such as about 55°C. In some embodiments, the preset temperature at which the composition described in (ii) is converted into gel state is about 50~60°C, such as about 55~60°C, such as about 55°C. In some embodiments, the preset temperature at which the reaction composition described in (iii) is converted into gel state is about 50~60°C, such as about 55~60°C, such as about 55°C. In some embodiments, the preset temperature at which the composition described in (iv) is converted into gel state is about 50~60°C, such as about 55~60°C, such as about 55°C. In some embodiments, the preset temperature at which the composition described in (v) is converted into gel state is about 35~45°C, such as about 37~45°C, about 37~42°C, such as about 37°C.

In some embodiments, in any of the above aspects, the kit may also comprise a washing solution.

In some embodiments, in any of the above aspects, the polymer having reversible thermogel property responds to a temperature change by changing from liquid state to gel state when the temperature rises. In some embodiments, the concentration of the polymer is about 0.5~30% (w/w), such as about 10~30% (w/w), about 15~25% (w/w), about 18~22% (w/w), such as about 20% (w/w). In some embodiments, the polymer has a gelling concentration of about 0.5% to 25%. In some embodiments, the polymer has a gelling temperature of about 10~65°C. In some embodiments, the polymer is a block copolymer, a graft copolymer, or a homopolymer. In some embodiments, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxypolyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA).

The present invention also relates to a use of the kit described in any of the above aspects or any of the compositions in the kit for sequencing. The present invention also relates to a sequencing method, which comprises using the kit described in any of the above aspects or any of the compositions in the kit.

### Beneficial effects of the invention

The present application provides a hydrogel-based sequencing method, which not only supports the traditional Flowcell-based sequencing system, but also supports the open sequencing system and can support large-size sequencing chips. The method can reduce reagent loss and avoid drying and crystallization damage to the target nucleic acid molecules on the surface of the slide; and does not require complex fluids and temperature control; it can also effectively reduce the amount of reagents used, thereby achieving single use and reducing costs. The method of the present application has broad application prospects.

### Specific Models for Carrying Out the Invention

The following will describe the embodiments of the present invention in detail in conjunction with the examples, but those skilled in the art will understand that the following examples are only used to illustrate the present invention and should not be regarded as limiting the scope of the present invention. If the specific conditions were not specified in the examples, they were carried out according to conventional conditions or conditions recommended by the manufacturer. The reagents or instruments used without indicating the manufacturer were all conventional products that could be purchased commercially.

### Preparation example:

### I. Reagents:

| Name | Company | Origin |
|---|---|---|
| Pluronic F-127 (Cat. No. P2443) | Sigma-Aldrich | USA |
| Methocel A15C (Cat. No. 64625) | Sigma-Aldrich | USA |
| Deionized formamide | Sangon | China |
| DIPSEQ-T1 sequencing slide | MGI | China |
| *E.coli* standard library | MGI | China |
| DNB preparation kit | MGI | China |
| DNB loading kit | MGI | China |
| Regeneration reagent | MGI | China |
| Regeneration buffer | MGI | China |
| DIPSEQ sequencing reagent | MGI | China |
| cPAS sequencing enzyme | MGI | China |
| dNTP mix | MGI | China |
| dNTP mix II | MGI | China |
| Wash buffer 2 | MGI | China |
| Sequencing primer 1 | MGI | China |
| Barcode sequencing primer 1 | MGI | China |
| Scanning reagent | MGI | China |

### II. Reagent preparation steps:

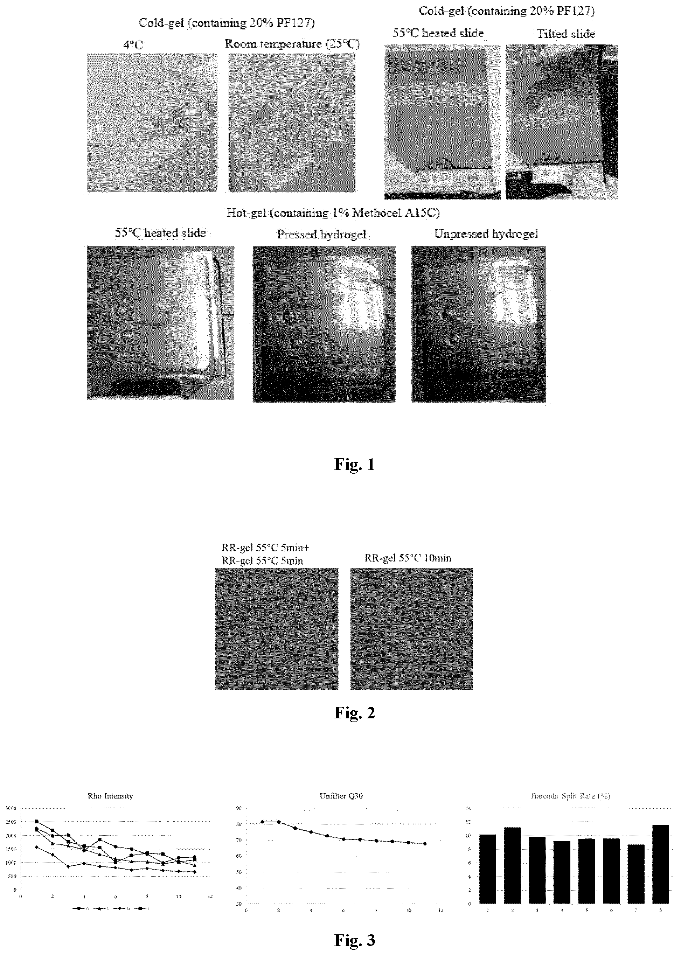

1. Preparation of RR-gel reagent: 37.5g of Pluronic F-127 was weighed, dissolved in 150mL of regeneration reagent (RR) to obtain RR-gel reagent (containing 20% (w/w) PF127), and stored at 4°C. Alternatively, 1.515g of Methocel A15C was weighed, dissolved in 150mL of regeneration reagent (RR) to obtain RR-gel reagent (containing 1% (w/w) Methocel A15C), and stored at 4°C.
2. Preparation of hot-gel reagent and cold-gel reagent: 37.5g of Pluronic F-127 was weighed, dissolved in 150mL of sequencing reagent, and stored at 4°C. 3mL of dNTP mix (carrying fluorescent label) and 3mL of sequencing enzyme were added to 144mL of the above-prepared sequencing reagent (containing 20% (w/w) PF127) to obtain hot-gel reagent, and stored at 4°C. 6mL of dNTP mix II (not carrying fluorescent label) and 3mL of sequencing enzyme were added to 141mL of the sequencing reagent prepared above (containing 20% (w/w) PF127) to obtain cold-gel reagent, and stored at 4°C.
   Alternatively, 1.515g of Methocel A15C was weighed and dissolved in 150mL of sequencing reagent. 3mL of dNTP mix (carrying fluorescent label) and 3mL of sequencing enzyme were added to 144mL of the sequencing reagent prepared above (containing 1% (w/w) Methocel A15C) to obtain hot-gel reagent, and stored at 4°C. 6mL of dNTP mix II (not carrying fluorescent label) and 3mL of sequencing enzyme were added to 141mL of the sequencing reagent prepared above (containing 1% (w/w) Methocel A15C) to obtain cold-gel reagent, and stored at 4°C.
3. Preparation of hot reagent and cold reagent: 3mL of dNTP mix (carrying fluorescent label) and 3mL of sequencing enzyme were added to 144mL of sequencing reagent (not containing PF127 and Methocel A15C) to obtain hot reagent, and stored at 4°C. 6mL of dNTP mix II (not carrying fluorescent label) and 3mL of sequencing enzyme were added to 141mL of sequencing reagent (not containing PF127 and Methocel A15C) to obtain cold reagent, and stored at 4°C.
4. Result analysis: As shown in Fig. 1, the reagent containing 20% (w/w) PF127 was in solution state at 4°C and in hydrogel state at room temperature. The reagent containing 20% (w/w) PF127 was added dropwise to the slide, and a hydrogel was formed after heating. The hydrogel did not flow when the slide was tilted. Similarly, the reagent containing 1% (w/w) Methocel A15C was added dropwise to the slide, and a hydrogel was formed after heating. The hydrogel had a certain elasticity.

### III. Sequencing cycle steps:

1. DNBs were prepared using DNB preparation kit, DIPSEQ-T1 sequencing slide and DNB loading reagent were used for DNB loading, and the sequencing slide underwent loading was stored in Wash buffer 2.
2. The sequencing slide was rinsed with the sequencing reagent, then added dropwise with hot-gel under low temperature conditions, and placed on a 55°C heating plate to form a hydrogel. After reacting for 1~3min or 5~10min, it was cooled down to 20°C or placed horizontally in a 4°C environment or dipped in 4°C Wash buffer 2. After the hydrogel was converted into a solution, the reagent was discarded, and rinsing was performed 3 times with Wash buffer 2.
3. The cold-gel was added dropwise under low temperature conditions and placed on a 55°C heating plate to form a hydrogel. After reacting for 1~3min or 5~10min, it was cooled down to 20°C or placed horizontally in a 4°C environment or dipped in 4°C Wash buffer 2. After the hydrogel was converted into a solution, the reagent was discarded, and rinsing was performed 3 times with Wash buffer 2.
4. A sequencer was used to photograph and basecall the sequencing slide to complete Cycle 1 sequencing.
5. The sequencing slide was rinsing with the regeneration buffer, then added dropwise with RR-gel under low temperature conditions and placed on a 55°C heating plate to form a hydrogel. After reacting for 1~3min or 5~10min, it was cooled to 20°C or placed horizontally in a 4°C environment or dipped in 4°C Wash buffer 2. After the hydrogel was converted into a solution, the reagent was discarded, and rinsing was performed 3 times with Wash buffer 2.
6. Steps 2, 3, 4, and 5 were repeated to complete multiple sequencing cycles.

### Example 1: Barcode sequencing with RR-gel reagent

1. DNB was prepared using DNB preparation kit, DIPSEQ-T1 sequencing slide and DNB loading reagent were used to perform DNB loading, hybridization of first strand of barcode sequencing primer was carried out, and the sequencing slide underwent loading was stored in Wash buffer 2.
2. The sequencing slide was rinsed with sequencing reagent, then added dropwise with 3mL of hot reagent under low temperature, and placed on a 55°C heating plate, the reagent was discarded after 3min of reaction, and the sequencing slide was rinsed 3 times with Wash buffer 2.
3. 3mL of cold reagent was added dropwise under low temperature, and placed on a 55°C heating plate, the reagent was discarded after 3min of reaction, and the sequencing slide was rinsed 3 times with Wash buffer 2.
4. The sequencing type was set to SE1+barcode10. A sequencer was used to photograph and basecall the sequencing slide to complete Cycle 1 sequencing.
5. The sequencing slide in step 4 was photographed and basecalled again to complete Cycle 2 sequencing.
6. The sequencing slide was rinsed with regeneration buffer, then added dropwise with RR-gel (containing 20% (w/w) PF127) under low temperature, and placed on a 55°C heating plate to form a hydrogel. After reacting for 10min, it was placed horizontally in a 4°C environment or dipped in 4°C Wash buffer 2. After the hydrogel was converted into a solution, the reagent was discarded, and rinsing was performed 3 times with Wash buffer 2.
7. Steps 2, 3, 4, and 6 were repeated to complete Cycle 3 to Cycle 11. A total of 11 sequencing cycles were completed and the sequencing was completed.
8. Result analysis: As shown in Fig. 2, RR-gel (containing 20% (w/w) PF127) was reacted for 5 minutes, and then RR-gel (containing 20% (w/w) PF127) was added again and reacted for 5 minutes to complete the excision; the removal effect of single RR-gel (containing 20% (w/w) PF127) reaction for 10 minutes was good, and similar to the efficiency of two rounds of RR-gel reaction. As shown in Fig. 3, SE1+barcode10 sequencing could be completed using RR-gel, the splitting rate of 441 fovs was 79%, and the splitting rate of single fov was 81%.

### Example 2: Read1 sequencing with Hot-gel reagent

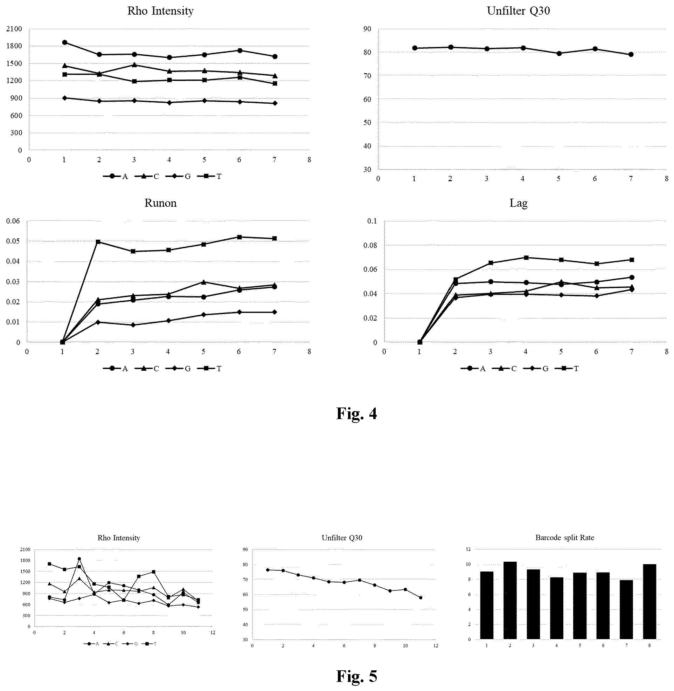

1. DNB was prepared using DNB preparation kit, DIPSEQ-T1 sequencing slide and DNB loading reagent were used to perform DNB loading, hybridization of first strand of sequencing primer 1 was carried out, and the sequencing slide underwent loading was stored in Wash buffer 2.
2. The sequencing slide was rinsed with sequencing reagent, then added dropwise with hot-gel (containing 1% (w/w) Methocel A15C) under low temperature conditions, and placed on a 55°C heating plate to form a hydrogel. After reacting for 3 minutes, it was cooled to 20°C and placed horizontally or dipped in Wash buffer 2. After the hydrogel is converted into a solution, the reagent was discarded, and rinsing was carried out 3 times with Wash buffer 2.
3. 3mL of cold reagent was added under low temperature conditions, and placed on a 55°C heating plate, the reagent was discarded after reacting for 3 minutes, and the sequencing slide was rinsed 3 times with Wash buffer 2.
4. The sequencing type was set to SE7. A sequencer was used to photograph and basecall the sequencing slide to complete Cycle 1 sequencing.
5. The sequencing slide was rinsed with regeneration buffer, then added dropwise with RR under low temperature conditions, and placed on a 55°C heating plate, the reagent was discarded after reacting for 5 minutes, and rinsing was performed 3 times with Wash buffer 2.
6. Steps 2, 3, 4, and 5 were repeated to complete Cycle 2 to Cycle 8. A total of 8 sequencing cycles were completed (the 8^{th} cycle was the calibration cycle), and the sequencing was completed.
7. Result analysis: As shown in Fig. 4, SE7 sequencing could be completed using hot-gel, the signal intensity was relatively stable, and Unfilter Q30 remained at about 80%.

### Example 3: Barcode sequencing with Hot-gel, Cold-gel and RR-gel reagents

1. DNB was prepared using DNB preparation kit, DIPSEQ-T1 sequencing slide and DNB loading reagent were used to perform DNB loading, hybridization of first strand of barcode sequencing primer was carried out, and the sequencing slide underwent loading was stored in Wash buffer 2.
2. The sequencing slide was rinsed with sequencing reagent, then added dropwise with hot-gel (containing 20% (w/w) PF127) at low temperature, and placed on a 55°C heating plate to form a hydrogel. After reacting for 8 minutes, it was placed horizontally in a 4°C environment or dipped in 4°C Wash buffer 2. After the hydrogel was converted into a solution, the reagent was discarded, and rinsing was performed 3 times with Wash buffer 2.
3. Cold-gel (containing 20% (w/w) PF127) was added dropwise at low temperature, and placed on a 55°C heating plate to form a hydrogel. After reacting for 8 minutes, it was placed horizontally in a 4°C environment or dipped in 4°C Wash buffer 2. After the hydrogel was converted into a solution, the reagent was discared, and rinsing was performed 3 times with Wash buffer 2.
4. The sequencing type was set to SE1+barcode10. A sequencer was used to photograph and basecall the sequencing slide to complete Cycle 1 sequencing.
5. The sequencing slide in step 4 was photographed and basecalled again to complete Cycle 2 sequencing.
6. The sequencing slide was rinsed with regeneration buffer, then added dropwise with RR-gel (containing 20% (w/w) PF127) under low temperature conditions and placed on a 55°C heating plate to form a hydrogel. After reacting for 5 minutes, it was placed horizontally in a 4°C environment or dipped in 4°C Wash buffer 2. After the hydrogel was converted into a solution, the reagent was discarded, and rinsing was performed 3 times with Wash buffer 2.
7. Steps 2, 3, 4, and 6 were repeated to complete Cycle 3 to Cycle 11. A total of 11 sequencing cycles were completed, and the sequencing was completed.
8. Result analysis: As shown in Fig. 5, hot-gel, cold-gel, and RR-gel could complete SE1+barcode10 sequencing, and the splitting rate of 36 fovs was 72.25%, and the splitting rate of single fov was 75.53%.

### Example 4: Read1 sequencing with hot-gel, cold-gel and RR-gel reagents

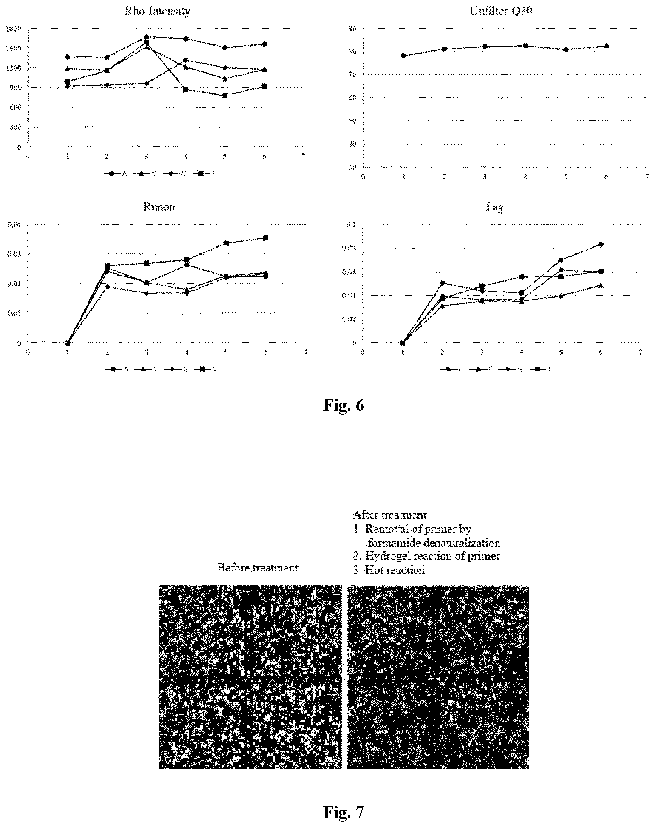

1. DNB was prepared using DNB preparation kit, DIPSEQ-T1 sequencing slide and DNB loading reagent were used to perform DNB loading, hybridization of first strand of sequencing primer was carried out, and the sequencing slide underwent loading was stored in Wash buffer 2.
2. The sequencing slide was rinsed with sequencing reagent, then added dropwise with hot-gel (containing 20% (w/w) PF127) under low temperature conditions, and placed on a 55°C heating plate to form a hydrogel. After reacting for 8 minutes, it was placed horizontally in a 4°C environment or dipped in 4°C Wash buffer 2. After the hydrogel was converted into a solution, the reagent was discarded, and rinsing was performed 3 three times with Wash buffer 2.
3. Cold-gel (containing 20% (w/w) PF127) was added dropwise under low temperature conditions and placed on a 55°C heating plate to form a hydrogel. After reacting for 8 minutes, it was placed horizontally in a 4°C environment or dipped in 4°C Wash buffer 2. After the hydrogel was converted into a solution, the reagent was discarded, and rinsing was performed 3 times with Wash buffer 2.
4. The sequencing type was set to SE6. A sequencer was used to photograph and basecall the sequencing slide to complete Cycle 1 sequencing.
5. The sequencing slide was rinsed with regeneration buffer, then added dropwise with RR-gel (containing 20% (w/w) PF127) under low temperature conditions, and placed on a 55°C heating plate to form a hydrogel. After reacting for 5 minutes, it was placed horizontally in a 4°C environment or dipped in 4°C Wash buffer 2. After the hydrogel was converted into a solution, the reagent was discarded, and rinsing was performed 3 times with Wash buffer 2.
6. Steps 2, 3, 4, and 5 were repeated to complete Cycle 2 to Cycle 7. A total of 7 sequencing cycles were completed (the 7^{th} cycle was the calibration cycle), and the sequencing was completed.
7. Result analysis: As shown in Fig. 6, hot-gel, cold-gel and RR-gel could complete SE6 sequencing, the signal intensity was relatively stable, and the Unfilter Q30 was maintained at about 80%.

### Example 5: Primer-gel primer hybridization reaction

1. 5g of Pluronic F-127 was weighed, dissolved in 20mL of Sequencing primer 1 solution, to obtain Primer-gel reagent, and stored at 4°C.
2. DNB was prepared using DNB preparation kit, DIPSEQ-T1 sequencing slide and DNB loading reagent were used to perform DNB loading, hybridization of first strand of sequencing primer was carried out, and the sequencing slide underwent loading was stored in Wash buffer 2.
3. A sequencer was used to photograph pictures and basecall the sequencing slide.
4. The sequencing slide was rinsed with Wash buffer 2, then added dropwise with 3mL of deionized formamide, placed on a 37°C heating plate for reaction for 10min to remove the first strand of sequencing primer. The formamide was discarded, rinsing was performed 3 times with Wash buffer 2, Primer-gel reagent was added dropwise under low temperature, placed on a 37°C heating plate to form a hydrogel. After reacting for 20 minutes, it was placed horizontally in a 4°C environment or dipped in 4°C Wash buffer 2. After the hydrogel was converted into a solution, the reagent was discarded, and rinsing was performed 3 times with Wash buffer 2.
5. A sequencer was used again to photograph and basecall the sequencing slide.
6. Result analysis: As shown in Fig. 7, the primer in the reagent that formed the hydrogel could be hybridized to DNB.

Although the specific embodiments of the present invention have been described in detail, those skilled in the art will understand that various modifications and changes can be made to the details based on all the teachings that have been disclosed, and these changes are within the scope of protection of the present invention. The entirety of the present invention is given by the appended claims and any equivalents thereof.

## Claims

1. A method for sequencing a target nucleic acid molecule, comprising the following steps:
(1) contacting nucleotides having a label with the target nucleic acid molecule to allow the incorporation of a labeled nucleotide;
(2) detecting the label of the nucleotide as incorporated;
(3) removing the label;
wherein, at least one of steps (1) and (3) is performed in gel state;
optionally, the method comprises repeating the above steps in sequence to determine the sequence of the target nucleic acid molecule;
preferably, the incorporation of the nucleotide in step (1) results in formation of a phosphodiester bond;
preferably, step (3) allows a complementary chain of the target nucleic acid molecule to undergo an extension reaction.

2. The method according to claim 1, wherein the label is a fluorescent label.

3. The method according to claim 1, wherein the label is an affinity label, and the step (2) comprises: step (2a), adding a luciferase carrying an affinity label to perform an affinity reaction with the product of step (1); step (2b), adding a specific substrate to perform a catalytic reaction with the product of step (2a); step (2c), detecting the product of step (2b).

4. The method according to claim 3, wherein steps (2a) and (2b) are performed in gel state.

5. A method for sequencing a target nucleic acid molecule, comprising the following steps:
(1) contacting nucleotides with the target nucleic acid molecule to allow the incorporation of a nucleotide, wherein the nucleotides carry a blocking group;
(2) detecting the nucleotide as incorporated;
(3) removing the blocking group;
wherein, at least one of steps (1) and (2) is performed in gel state;
optionally, the method comprises repeating the above steps in sequence to determine the sequence of the target nucleic acid molecule;
preferably, the incorporation of the nucleotide in step (1) results in formation of a phosphodiester bond;
preferably, step (3) allows the complementary chain of the target nucleic acid molecule to undergo an extension reaction.

6. The method according to claim 5, wherein step (2) comprises: step (2a), adding an affinity reagent carrying a fluorescent label to perform an affinity reaction with the product of step (1); and step (2b), detecting the product of step (2a).

7. The method according to claim 6, wherein step (2a) is performed in gel state.

8. A method for sequencing a target nucleic acid molecule, comprising the following steps:
(1) contacting nucleotides having a label with the target nucleic acid molecule to allow the incorporation of a labeled nucleotide;
(2) detecting the label of the nucleotide as incorporated;
(3) removing the nucleotide as incorporated and the label it carries;
(4) contacting unlabeled nucleotides with the target nucleic acid molecule to allow the incorporation of an unlabeled nucleotide;
wherein, at least one of steps (1), (3) and (4) is performed in gel state;
optionally, the method comprises repeating the above steps in sequence to determine the sequence of the target nucleic acid molecule;
preferably, the nucleotide incorporation described in step (1) does not form a phosphodiester bond;
preferably, the incorporation of the nucleotide described in step (4) results in formation of a phosphodiester bond;
preferably, the nucleotide as incorporated in step (4) has a blocking group;
preferably, step (3) further comprises removing the blocking group remaining at the 3' end of the complementary chain of the target nucleic acid molecule, which allows the extension reaction of the complementary chain of the target nucleic acid molecule.

9. The method according to any one of claims 1 to 8, wherein step (1) is performed in gel state, and the method comprises the following steps:
(1a) contacting a reaction composition comprising nucleotides with the target nucleic acid molecule, wherein the nucleotides are labeled with a label or carry a blocking group, the reaction composition comprises a polymer having reversible thermogel property, and the reaction composition is in liquid state;
(1b) adjusting the reaction composition to a preset temperature to convert the reaction composition into gel state state, and performing an incorporation reaction to allow the incorporation of a nucleotide;
(1c) adjusting the temperature of the reaction composition to convert the reaction composition into liquid state.

10. The method according to claim 9, wherein after converting the reaction composition into liquid state in step (1c), the method further comprises: removing the reaction composition and then perform the detection reaction of step (2) or directly performing the detection reaction of step (2).

11. The method according to any one of claims 1 to 7, wherein step (3) is performed in gel state, and the method comprises the following steps:
(3a) contacting a reaction composition comprising a removal agent with the product of the previous step, wherein the reaction composition further comprises a polymer having reversible thermogel property, and the reaction composition is in liquid state;
(3b) adjusting the reaction composition to a preset temperature to convert the reaction composition into gel state, and performing an excision reaction to allow the removal of the label or blocking group on the nucleotide as incorporated;
(3c) adjusting the temperature of the reaction composition to convert the reaction composition into liquid state, and removing the reaction composition.

12. The method according to claim 8, wherein step (3) is performed in solution state, and the method comprises: removing the incorporated labeled nucleotide under an appropriate condition using an elution agent; preferably, removing the remaining blocking group.

13. The method according to claim 8, wherein step (3) is performed in gel state, and the method comprises the following steps:
(3a) contacting a reaction composition comprising an elution reagent with the product of the previous step, wherein the reaction composition further comprises a polymer having reversible thermogel property, and the reaction composition is in liquid state;
(3b) adjusting the reaction composition to a preset temperature to convert the reaction composition into gel state, and performing an elution reaction to allow removal of the nucleotide as incorporated and removal of the remaining blocking group;
(3c) adjusting the temperature of the reaction composition to convert the reaction composition into liquid state, and removing the reaction composition.

14. The method according to claim 8, wherein step (4) is performed in gel state, and the method comprises the following steps:
(4a) contacting a reaction composition comprising unlabeled nucleotides with the target nucleic acid molecule, wherein the reaction composition comprises a polymer having reversible thermogel property, and the reaction composition is in liquid state; preferably, the unlabeled nucleotides have a blocking group;
(4b) adjusting the reaction composition to a preset temperature to convert the reaction composition into gel state, and performing a polymerization reaction to allow the incorporation of an unlabeled nucleotide;
(4c) adjusting the temperature of the reaction composition to convert the reaction composition into liquid state, and optionally removing the reaction composition.

15. The method according to any one of claims 1 to 4 and 8, wherein the labeled nucleotide described in step (1) further comprises an independent blocking group; or, the label contained in the labeled nucleotide can be used as a blocking group.

16. The method according to any one of claims 1 to 4 and 8, wherein before, during or after any step before step (3) (for example, after step (1) and before step (2)), the method further comprises: a step of contacting unlabeled nucleotides with the target nucleic acid molecule, wherein the unlabeled nucleotides carry a blocking group; the step is optionally performed in gel state.

17. The method according to claim 16, wherein after step (1) and before step (2), the method comprises the following steps:
(i) contacting a reaction composition comprising unlabeled nucleotides with the target nucleic acid molecule, wherein the unlabeled nucleotides carry a blocking group, the reaction composition comprises a polymer having reversible thermogel property, and the reaction composition is in liquid state;
(ii) adjusting the reaction composition to a preset temperature to convert the reaction composition into gel state, and performing an incorporation reaction to allow the incorporation of nucleotide;
(iii) adjusting the temperature of the reaction composition to convert the reaction composition into liquid state, and removing the reaction composition.

18. The method according to any one of claims 1 to 17, wherein, before step (1), the method further comprises: a step of contacting a sequencing primer with the target nucleic acid molecule to allow hybridization; the step is optionally performed in gel state;
preferably, before step (1), the method comprises the following steps:
(i) contacting a reaction composition comprising a sequencing primer with the target nucleic acid molecule, wherein the reaction composition comprises a polymer having reversible thermogel property, and the reaction composition is in liquid state;
(ii) adjusting the reaction composition to a preset temperature to convert the reaction composition into gel state so that the sequencing primer hybridizes to the target nucleic acid molecule; preferably, the preset temperature allows the primer hybridization to occur;
(iii) adjusting the temperature of the reaction composition to convert the reaction composition into liquid state, and removing the reaction composition.

19. The method according to any one of claims 1 to 18, wherein the polymer having reversible thermogel property responds to a temperature change by changing from liquid state to gel state when the temperature rises;
preferably, the concentration of the polymer is about 0.5~30% (w/w), such as about 10~30% (w/w), about 15~25% (w/w), about 18~22% (w/w), such as about 20% (w/w);
preferably, the polymer has a gelling concentration of about 0.5% to 25% (w/w);
preferably, the polymer has a gelling temperature of about 10~65°C;
preferably, the polymer is a block copolymer, a graft copolymer or a homopolymer;
preferably, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxy polyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA).

20. The method according to any one of claims 1 to 19, wherein:
(a) the temperature at which the gel state is converted into the liquid state is about 0~30°C (e.g., about 0~10°C, such as about 2~8°C, such as about 4°C; such as about 20~30°C, such as about 20~25°C, such as about 20°C or about 25°C);
(b) the preset temperature at which the reaction composition comprising nucleotides (e.g., labeled nucleotides, unlabeled nucleotides, or unlabeled nucleotides with a blocking group) is converted into gel state is about 50~60°C, such as about 55~60°C, such as about 55°C;
(c) the preset temperature at which the reaction composition comprising a removal reagent or an elution reagent is converted into gel state is about 50~60°C, such as about 55~60°C, such as about 55°C; and/or,
(d) the preset temperature at which the reaction composition comprising a sequencing primer is converted into gel state is about 35~45°C, such as about 37~45°C, about 37~42°C, such as about 37°C.

21. The method according to any one of claims 1 to 20, wherein the target nucleic acid molecule is fixed to a solid phase support, such as a chip.

22. The method according to any one of claims 1 to 21, wherein the method is an open sequencing method;
preferably, the target nucleic acid molecule is fixed to the surface of an open sequencing slide;
preferably, the method comprises allowing the open sequencing slide with the target nucleic acid molecule fixed thereon to contact with or dip into one or more sequencing reagents required for the sequencing reaction, respectively, and the one or more sequencing reagents are each placed in a separate reaction container.

23. The method according to any one of claims 1 to 21, wherein the method is a Flowcell sequencing method;
preferably, the target nucleic acid molecule is fixed to the surface of a flow cell;
preferably, the method comprises introducing one or more sequencing reagents required for the sequencing reaction into a flow cell with the target nucleic acid molecule fixed thereon.

24. A kit, which comprises:
(a) a composition comprising labeled nucleotides and a polymer having reversible thermogel property; and/or,
(b) a composition comprising a removal agent and a polymer having reversible thermogel property;
preferably, the kit further comprises: (c) a composition comprising unlabeled nucleotides and a polymer having reversible thermogel property, wherein the unlabeled nucleotides carry a blocking group;
preferably, the kit further comprises: (d) a composition comprising a sequencing primer and a polymer having reversible thermogel property;
preferably, the polymer having reversible thermogel property described in any one of (a) to (d) may be the same or different;
preferably, the kit further comprises an additional reagent required for sequencing, such as a washing solution.

25. A kit, which comprises:
(1) a composition comprising nucleotides and a polymer having reversible thermogel property, wherein the nucleotides carry a blocking group; and/or,
(2) a composition comprising an affinity agent carrying a fluorescent label and a polymer having reversible thermogel property;
preferably, the kit further comprises: (3) a composition comprising a removal agent and a polymer having reversible thermogel property;
preferably, the kit further comprises: (4) a composition comprising a sequencing primer and a polymer having reversible thermogel property;
preferably, the polymer having reversible thermogel property described any one of (1) to (4) may be the same or different;
preferably, the kit further comprises an additional reagent required for sequencing, such as a washing solution.

26. A kit, which comprises:
(i) a composition comprising labeled nucleotides and a polymer having reversible thermogel property;
(ii) a composition comprising an elution reagent and a polymer having reversible thermogel property; and/or,
(iii) a composition comprising unlabeled nucleotides and a polymer having reversible thermogel property; preferably, the unlabeled nucleotides carry a blocking group;
preferably, the kit further comprises: (iv) a composition comprising unlabeled nucleotides and a polymer having reversible thermogel property, wherein the unlabeled nucleotides carry a blocking group;
preferably, the kit further comprises: (v) a composition comprising a sequencing primer and a polymer having reversible thermogel property;
preferably, the polymer having reversible thermogel property described in any one of (i) to (v) may be the same or different;
preferably, the kit further comprises an additional reagent required for sequencing, such as a washing solution.

27. A kit according to any one of claims 24 to 26, wherein the polymer having reversible thermogel property responds to a temperature change by changing from liquid state to gel state when the temperature rises;
preferably, the concentration of the polymer is about 0.5~30% (w/w), such as about 10~30% (w/w), about 15~25% (w/w), about 18~22% (w/w), such as about 20% (w/w);
preferably, the polymer has a gelling concentration of about 0.5% to 25%;
preferably, the polymer has a gelling temperature of about 10~65°C;
preferably, the polymer is a block copolymer, a graft copolymer or a homopolymer;
preferably, the polymer is selected from the group consisting of Pluronic block polymer (e.g., Pluronic F127), Tetronic block polymer, hydroxypropyl methylcellulose, methylcellulose (e.g., Methocel A15C, Methocel A15 LV), methoxy polyethylene glycol-block-poly(ε-caprolactone) (mPEG-PCL), poly(N-isopropylacrylamide-co-methacrylic acid) (pNIPAm-co-AA), poly(lactic acid-co-hydroxylactic acid)-polyethylene glycol-poly(lactic acid-co-hydroxylactic acid) (PLGA-PEG-PLGA).

28. The kit according to any one of claims 24 to 27, wherein:
the temperature at which the composition described in any one of (a) to (d), (1) to (4), (i) to (v) is converted from gel state to liquid state is about 0~30°C (e.g., about 0~10°C, such as about 2~8°C, such as about 4°C; such as about 20~30°C, such as about 20~25°C, such as about 20°C or about 25°C);
the preset temperature at which the reaction composition described in (a), (1) or (i) is converted into gel state is about 50~60°C, such as about 55~60°C, such as about 55°C;
the preset temperature at which the reaction composition described in (2) is converted into gel state is about 30~40°C, such as about 30~37°C, about 32~37°C, about 35 ~37°C, such as about 35°C;
the preset temperature at which the reaction composition described in (b), (3) or (ii) is converted into gel state is about 50~60°C, such as about 55~60°C, such as about 55°C;
the preset temperature at which the reaction composition described in (iii) is converted into gel state is about 50~60°C, such as about 55~60°C, such as about 55°C;
the preset temperature at which the reaction composition described in (c) or (iv) is converted into gel state is about 50~60°C, such as about 55~60°C, such as about 55°C; and/or,
the preset temperature at which the reaction composition described in (d), (4) or (v) is converted into gel state is about 35~45°C, such as about 37~45°C, about 37~42°C, such as about 37°C.
